# EUROPÄISCHE PATENTANMELDUNG

(11) **EP 1 130 103 A2**
(43) Veröffentlichungstag der Anmeldung: **05.09.2001**
(21) Anmeldenummer: 00125888.8
(22) Anmeldetag: 27.11.2000
(51) Int. Cl.: C12N 15/61, C12N 15/11, C12N 9/90, C07K 16/40, C12P 7/64, C12N 1/10

(54) **Neue Nukleinsäure aus Tetrahymena kodierend für eine Trieterpenoid-Cyclase, ihre Herstellung und Verwendung**

(30) Priorität: 01.12.1999 DE 19957889
(71) Anmelder: Celanese Ventures GmbH, 65926 Frankfurt am Main (DE)
(72) Erfinder: Rüsing, Matthias, Dr., 50935 Köln (DE); Schweins, Thomas, Dr., 40545 Düsseldorf (DE); Dresler, Petra, 65830 Kriftel (DE); Stock, Wolfgang, Dr., 40227 Düsseldorf (DE); Kiy, Thomas, Dr., 65929 Frankfurt am Main (DE)
(74) Vertreter: Luderschmidt, Schüler & Partner GbR

(57) **Zusammenfassung**

Die vorliegende Erfindung betrifft Nukleinsäuren aus Tetrahymena die für eine ciliatenspezifische Triterpenoid-Cyclase kodieren. Die erfindungsgemäßen Nukleotidsequenzen und die daraus abgeleitete Polypeptidsequenz zeigen dabei eine überraschend geringe Sequenzidentität zu bekannten Isoprenoid-Cyclasen. Die Erfindung betrifft ferner die Verwendung der Nukleinsäuren für eine kontrollierte Expression in einem Wirt sowie deren gezielte Ausschaltung (Knock-out) oder Reprimierung. Durch die dadurch bewirkte Kontrolle der Expression der Triterpenoid-Cyclase ist es möglich, den Gehalt an mehrfach ungesättigten Fettsäuren im Wirt zu modifizieren und anzureichern.

SEQUENZPROTOKOLL

<110> Celanese Ventures GmbH

<120> Neue Nukleinsäure aus Tetrahymena kodierend für eine
      Triterpenoid-Cyclase, ihre Herstellung und Verwendung

<130> AX99056WO

<140>
<141>

<150> DE 199 57 889.3-41
<151> 1999-12-01

<160> 13

<170> PatentIn Ver. 2.1

<210> 1
<211> 22
<212> DNA
<213> Künstliche Sequenz

<220>
<223> Beschreibung der künstlichen Sequenz:Primer

<400> 1
ggttcntggt ayggtagatg gg                                          22


<210> 2
<211> 18
<212> DNA
<213> Künstliche Sequenz

<220>
<223> Beschreibung der künstlichen Sequenz:Primer

<400> 2
ttcaccccaa ccaccatc                                               18


<210> 3
<211> 39
<212> DNA
<213> Künstliche Sequenz

<220>
<223> Beschreibung der künstlichen Sequenz:Primer

<400> 3
gaccacgcgt atcgatgtcg actttttttt tttttttv                                39

<210> 4
<211> 22
<212> DNA
<213> Künstliche Sequenz

<220>
<223> Beschreibung der künstlichen Sequenz:Primer

<400> 4
ctgttggagc tgttgtacca gg                                                 22

<210> 5
<211> 25
<212> DNA
<213> Künstliche Sequenz

<220>
<223> Beschreibung der künstlichen Sequenz:Primer

<400> 5
cgtaattgac tcttgctaaa cctgg                                              25

<210> 6
<211> 27
<212> DNA
<213> Künstliche Sequenz

<220>
<223> Beschreibung der künstlichen Sequenz:Primer

<400> 6
gctaaaactc tttcatacat gaagaag                                            27

<210> 7
<211> 32
<212> DNA
<213> Künstliche Sequenz

<220>
<223> Beschreibung der künstlichen Sequenz:Primer

<400> 7
ctctttcata catgcataag atactcatag gc                                    32

<210> 8
<211> 35
<212> DNA
<213> Künstliche Sequenz

<220>
<223> Beschreibung der künstlichen Sequenz:Primer

<400> 8
ggcttggatc ctcaaatatt ttatttttat acagg                                 35

<210> 9
<211> 30
<212> DNA
<213> Künstliche Sequenz

<220>
<223> Beschreibung der künstlichen Sequenz:Primer

<400> 9
aaaaataaaa aagtttgaaa aaaaaccttc                                       30

<210> 10
<211> 22
<212> DNA
<213> Künstliche Sequenz

<220>
<223> Beschreibung der künstlichen Sequenz:Primer

<400> 10
gtttagctga ccgattcagt tc                                               22

<210> 11
<211> 2072
<212> DNA
<213> Tetrahymena thermophila

<400> 11
aaaaaagcta aaactctttc atacatgaag aagatactca taggcttaat tataggtctc 60
tttttattct caagcgttaa tgccagcgtt aatctcactg aagtctaaaa tgccatctct 120
atctagcaag gcattaattg ggcagaagta cacaacaata cttggtacta tcctccttac 180
ttaggcgaaa tgtttatcag tgaatactac ttcgagttac tcgtcttgaa ttggactcat 240
aaatctgctt tcaacgctac atactttaca gaacgtctcc tctagactta attcgaagat 300
ggttcatggg agcaagtcag agaacaaaat cttgaaactg gttagttaga tgctactgtc 360
tttaactact ggtacttaaa gtctattaac aacaatccta aaattgaagc tgctctataa 420
aaggctagaa aatggatagt tgcttagggt ggtattgaag caactcaaac aatgaccaag 480
tttaagttag cagccttcgg ttaatacagt tgggaagatt tatggtatgt cccattgttc 540
atcttcaagt agaatggaat tttcaaatat acctacgtta aggatattgt tgcataatgg 600
gtctatccac atttaactgc cttagcttat ttgcgttact aaagaactgt tttcaatgtt 660
cctgttgctg atttgagaga gctctggatc aattacccta agaacggtat taaaatcagt 720
ccaagagaat actctacact taatcctgat agcgatctct tgatcttaat ggacgaaatc 780
ttcaaactta aacaacctct tggaagtttc ggtgcctaca ctatttcaac cctcttgact 840
ttaatgtcct tcaaagactt ttagtcaaag caccctcatc tataccaaaa cgaaatacaa 900
aaggcttacg aagacggata ctatttcgtt gagtttaact actttaactt tagagaagct 960
tatcacggct ctttggatga tggtagatgg tgggatacca ttcttattag ttgggctatg 1020
cttgaaagtg gctaagataa agaaagaatc ttccctatcg tataaaatat ggtcaaagaa 1080
ggtctttaac ctaaaaaagg tataggttat ggatatgatt tcgaatatgc tcctgacact 1140
gatgacactg gattacttct cgttgttatg agttactaca aagaagcctt ctaaaagtaa 1200
atccctgaaa ctattgaatg gcttttctct atgcaaaatg acgatggtgg ctatccagct 1260
tttgacaaag gtaaaaatga agacaattta ttgttcaagt ttgccttcaa tatggctggt 1320
attgctaact cagctgaaat cttcgatccc tcatgtcctg atattactgg tcacatcatg 1380
gaaggattgg gtgagtttgg atatcaagct aatcatcctt agatttaaaa tatgattaaa 1440
tatcaaagaa agacttagaa caagtgggga tcttggtaag ctagatgggg tgtaaattac 1500
attatggctg ttggagctgt tgtaccaggt ttagcaagag tcaattacga cttaaatgaa 1560
cagtgggtac aaaatagtat aaattatttg cttaataaat aaaataaaga tggtggcttt 1620
ggtgaatgtg tcctttctta taatgatcct gaaaagtgga atggtatagg taaatctact 1680
gtcactcaaa cctcatgggg actattagct cttttagaag tttataatta aaatgaacaa 1740
attaagcatg ctgcagatag agctgcttag tatttattag attaattcaa aagagacgat 1800
aataccttct atgatcactc cacaatagga acaggtcaca gaggattact ctatttatag 1860
tacccctcat atgcacaatc attcccatta gtagctttaa atagatacta aaaaatatct 1920
caaggttaat atcacttctc caaaaatttg tacaatggta atggagaacc tgtataaaaa 1980
taaaatattt gaaaattcaa taaactgtat tttacatttt aaatttattt gtattttttt 2040
aagttatttt ttcataaaat aaaaaaaaaa aa 2072

<210> 12
<211> 655
<212> PRT
<213> Tetrahymena thermophila

<400> 12
Met Lys Lys Ile Leu Ile Gly Leu Ile Ile Gly Leu Phe Leu Phe Ser
1               5                   10                  15

Ser Val Asn Ala Ser Val Asn Leu Thr Glu Val Gln Asn Ala Ile Ser

            20                  25                  30

Ile Gln Gln Gly Ile Asn Trp Ala Glu Val His Asn Asn Thr Trp Tyr
        35                  40                  45

Tyr Pro Pro Tyr Leu Gly Glu Met Phe Ile Ser Glu Tyr Tyr Phe Glu
    50                  55                  60

Leu Leu Val Leu Asn Trp Thr His Lys Ser Ala Phe Asn Ala Thr Tyr
65                  70                  75                  80

Phe Thr Glu Arg Leu Leu Gln Thr Gln Phe Glu Asp Gly Ser Trp Glu
                85                  90                  95

Gln Val Arg Glu Gln Asn Leu Glu Thr Gly Gln Leu Asp Ala Thr Val
            100                 105                 110

Phe Asn Tyr Trp Tyr Leu Lys Ser Ile Asn Asn Asn Pro Lys Ile Glu
        115                 120                 125

Ala Ala Leu Gln Lys Ala Arg Lys Trp Ile Val Ala Gln Gly Gly Ile
    130                 135                 140

Glu Ala Thr Gln Thr Met Thr Lys Phe Lys Leu Ala Ala Phe Gly Gln
145                 150                 155                 160

Tyr Ser Trp Glu Asp Leu Trp Tyr Val Pro Leu Phe Ile Phe Lys Gln
                165                 170                 175

Asn Gly Ile Phe Lys Tyr Thr Tyr Val Lys Asp Ile Val Ala Gln Trp
            180                 185                 190

Val Tyr Pro His Leu Thr Ala Leu Ala Tyr Leu Arg Tyr Gln Arg Thr
        195                 200                 205

Val Phe Asn Val Pro Val Ala Asp Leu Arg Glu Leu Trp Ile Asn Tyr
    210                 215                 220

Pro Lys Asn Gly Ile Lys Ile Ser Pro Arg Glu Tyr Ser Thr Leu Asn
225                 230                 235                 240

Pro Asp Ser Asp Leu Leu Ile Leu Met Asp Glu Ile Phe Lys Leu Lys
                245                 250                 255

Gln Pro Leu Gly Ser Phe Gly Ala Tyr Thr Ile Ser Thr Leu Leu Thr
            260                 265                 270

Leu Met Ser Phe Lys Asp Phe Gln Ser Lys His Pro His Leu Tyr Gln

        275                 280                 285

Asn Glu Ile Gln Lys Ala Tyr Glu Asp Gly Tyr Tyr Phe Val Glu Phe
    290                 295                 300

Asn Tyr Phe Asn Phe Arg Glu Ala Tyr His Gly Ser Leu Asp Asp Gly
305                 310                 315                 320

Arg Trp Trp Asp Thr Ile Leu Ile Ser Trp Ala Met Leu Glu Ser Gly
                325                 330                 335

Gln Asp Lys Glu Arg Ile Phe Pro Ile Val Gln Asn Met Val Lys Glu
            340                 345                 350

Gly Leu Gln Pro Lys Lys Gly Ile Gly Tyr Gly Tyr Asp Phe Glu Tyr
        355                 360                 365

Ala Pro Asp Thr Asp Asp Thr Gly Leu Leu Leu Val Val Met Ser Tyr
    370                 375                 380

Tyr Lys Glu Ala Phe Gln Lys Gln Ile Pro Glu Thr Ile Glu Trp Leu
385                 390                 395                 400

Phe Ser Met Gln Asn Asp Asp Gly Gly Tyr Pro Ala Phe Asp Lys Gly
                405                 410                 415

Lys Asn Glu Asp Asn Leu Leu Phe Lys Phe Ala Phe Asn Met Ala Gly
            420                 425                 430

Ile Ala Asn Ser Ala Glu Ile Phe Asp Pro Ser Cys Pro Asp Ile Thr
        435                 440                 445

Gly His Ile Met Glu Gly Leu Gly Glu Phe Gly Tyr Gln Ala Asn His
    450                 455                 460

Pro Gln Ile Gln Asn Met Ile Lys Tyr Gln Arg Lys Thr Gln Asn Lys
465                 470                 475                 480

Trp Gly Ser Trp Gln Ala Arg Trp Gly Val Asn Tyr Ile Met Ala Val
                485                 490                 495

Gly Ala Val Val Pro Gly Leu Ala Arg Val Asn Tyr Asp Leu Asn Glu
            500                 505                 510

Gln Trp Val Gln Asn Ser Ile Asn Tyr Leu Leu Asn Lys Gln Asn Lys
        515                 520                 525

Asp Gly Gly Phe Gly Glu Cys Val Leu Ser Tyr Asn Asp Pro Glu Lys

530                 535                 540

Trp Asn Gly Ile Gly Lys Ser Thr Val Thr Gln Thr Ser Trp Gly Leu
545                 550                 555                 560

Leu Ala Leu Leu Glu Val Tyr Asn Gln Asn Glu Gln Ile Lys His Ala
                565                 570                 575

Ala Asp Arg Ala Ala Gln Tyr Leu Leu Asp Gln Phe Lys Arg Asp Asp
            580                 585                 590

Asn Thr Phe Tyr Asp His Ser Thr Ile Gly Thr Gly His Arg Gly Leu
        595                 600                 605

Leu Tyr Leu Gln Tyr Pro Ser Tyr Ala Gln Ser Phe Pro Leu Val Ala
    610                 615                 620

Leu Asn Arg Tyr Gln Lys Ile Ser Gln Gly Gln Tyr His Phe Ser Lys
625                 630                 635                 640

Asn Leu Tyr Asn Gly Asn Gly Glu Pro Val Gln Lys Gln Asn Ile
                645                 650                 655

<210> 13
<211> 5046
<212> DNA
<213> Tetrahymena thermophila

<400> 13
gaattcatac atttttgata gttcagaaaa taattatttt aatttaattt tttaatcatt 60
ccctcataat ttaataataa ttaaaaatta agactgcata tattaaggca ctttactttt 120
ataaaattaa ttttctacat tataaaatac agaagtattt tattatatat atttttcaaa 180
gtaaatgatt agcttttatt gattaaaatt aatgtgtgat taataatgtt acaatagcaa 240
tataagaaat attagtaaat cattataaga aaatataaac aagatagcat tcatatgcaa 300
aattaatttc tagaaatagt attcaaaaat gaagttaaaa tcctaagtac caactaatct 360
ttttaacata acctcattat tacgaaaata ttattatttt tttagtcact gtttgctaaa 420
tcgtatgatc tttttataaa tctaaaaaaa caaagtaaaa tttaaatatg agtatggctt 480
gtttctaaat ctatttagtg aaagctaatt tcaatttata tgtatttaga gaagcattaa 540
gttaatagga gggggaaaaa cgatgaaaaa ttaaaagtta ttgataagaa ttatttgtaa 600
tattatgttg taagttaaga attaataata ttattaaagg aatagaaagt tcttttaata 660
atattattaa agaaaaattg atatgtttga ggtgatgtca tgacgaaatt acatattatc 720
atgaacaaat caatggaaaa attgactgag ctaaaataaa aattgacgta aagagtttta 780
agtgcgtgtt agatttgaaa aagttaagaa aaatgacatg aatactggga ctttaataat 840
attatatatg taaggaattt attatattaa attcgtttca agttaaattc aaatttggct 900
taatattgtt agcaattaat tgattgtata gtcaacctta gctttaaaaa ccaactctaa 960
cttcaaggtt ttaataatat tattaatcaa ctcataaatt agtaaataag aaaaaagcta 1020
aaactctttc atacatgaag aagatactca taggcttaat tataggtctc tttttattct 1080

caagcgttaa tgccagcgtt aatctcactg aagtctaaaa tgccatctct atctagcaag 1140
gcattaattg ggcagaagta cacaacaata cttggtacta tcctccttac ttaggcgaaa 1200
tgtttatcag tgaatactac ttcgagttac tcgtcttgaa ttggactcat aaatctgctt 1260
tcaacgctac atactttaca gaacgtctcc tctagactta attcgaagat ggttcatggg 1320
agcaagtcag agaacaaaat cttgaaactg gttagttaga tgctactgtc tttaactact 1380
ggtacttaaa gtctattaac aacaatccta aaattgaagc tgctctataa aaggctagaa 1440
aatggatagt tgcttagggt ggtattgaag caactcaaac aatgaccaag tttaagttag 1500
cagccttcgg ttaatacagg taaagtttct ttttcatcaa tattttagaa ataaacaatc 1560
aattttaaat tattctccca tattttgctc aaataataat ttctacttaa ataattagct 1620
tcaactgcaa atataaaaat gaattaattt attataaata aaagcagtaa atataagcaa 1680
atatactaat ttaattagct tattattctg ttaatattta aaagccattt tgactcaata 1740
gcttatttta ttttaaataa ttaaatagtt gggaagattt atggtatgtc ccattgttca 1800
tcttcaagta gaatggaatt ttcaaatata cctacgttaa ggatattgtt gcataatggg 1860
tctatccaca tttaactgcc ttagcttatt tgcgttacta aagaactgtt ttcaatgttc 1920
ctgttgctga tttgagagag ctctggatca attaccctaa taacggtatt aaaatcagtc 1980
caagagaata ctctacactt aatcctgata gcgatctctt gatcttaatg gacgaaatct 2040
tcaaacttaa acaacctctt ggaagtttcg gtgcctacac tatttcaacc ctcttgactt 2100
taatgtcctt caaagacttt tagtcaaagc accctcatct ataccaaaac gaaatacaaa 2160
aggcttacga agacggatac tatttcgttg agtttaacta ctttaacttt agagaagctt 2220
atcacggctc tttggatgat ggtagatggt gggataccat tcttattagt tgggctatgc 2280
ttgaaagtgg ctaagataaa gaaagaatct tccctatcgt ataaaatatg gtcaaagaag 2340
gtctttaacc taaaaaaggt ataggttatg gatatgattt cgaatatgct cctgacactg 2400
atgacactgg attacttctc gttgttatga gttactacaa agaagccttc taaaagtaaa 2460
tccctgaaac tattgaatgg cttttctcta tgcaaaatga cgatggtggc tatccagctt 2520
ttgacaaagg taatttaata ttgataattt attccatttc tttatttaat aaaaataaat 2580
cttttaatta tttcaattga aagatacatt taaataaaat tacaaatgta cttaaaataa 2640
atataatatt attaacactt ctactttatt ttaaaatagg taaaaatgaa gacaatttat 2700
tgttcaagtt tgccttcaat atggctggta ttgctaactc agctgaaatc ttcgatccct 2760
catgtcctga tattactggt cacatcatgg aaggattggg tgagtttgga tatcaagcta 2820
atcatcctta gatttaaaat atgattaaat atcaaagaaa gacttagaac aagtggggat 2880
cttggtaagc tagatggggt gtaaattaca ttatggctgt tggagctgtt gtaccaggtt 2940
tagcaagagt caattacgac ttaaatgaac agtgggtaca aaatagtata aattatttgc 3000
ttaataaata aaataaagat ggtggctttg gtgaatgtgt cctttcttat aatgatcctg 3060
aaaagtggaa tggtataggt aaatctactg tcactcaaac ctcatgggga ctattagctc 3120
ttttagaagt ttataattaa aatgaacaaa ttaagcatgc tgcagataga gctgcttagt 3180
atttattaga ttaattcaaa agagacgata ataccttcta tgatcactcc acaataggaa 3240
caggtcacag aggattactc tatttatagt acccctcata tgcacaatca ttcccattag 3300
tagctttaaa tagatactaa aaaatatctc aaggttaata tcacttctcc aaaaatttgt 3360
acaatggtaa tggagaacct gtataaaaat aaaatatttg aaaattcaat aaactgtatt 3420
ttacatttta aatttatttg tattttttta agttattttt tcataaaata ataaataaat 3480
aaatttaatt tcatttttta tgaatttatt aatacacaaa aattttaatt attaatttta 3540
aaaatcgcat ttattggttt atcaatattt taagtttaaa attattttca gcattttctt 3600
caatatcaaa attcatagtt ttgacatatt aaattattca atgagttttt tatttttgct 3660
tttgtgagta atcaatcttt tttctaaatt tatattgcat taataaacaa atttaagtta 3720
tccattatca cttataatta tttagcatct aaaaattaat gcaaaacttt tttgatgaat 3780
cgattttact aagaattttt atttgttaaa ataagacaaa tgtaatttaa ataaattaat 3840
tccctcttaa attggtattt atttgtataa atctaattca tttaagtaga aattataatt 3900
aataattaaa ttagtaaaat gttatgatat taaacaaata aatagtatat gaatattata 3960

ttttaatcac atctcaatta gtatgctttt cgctgaataa aaagagcctt taataaatag 4020
tagtataatt tctttaaaat atacaatatt tttgaattaa ttggatttta aataaataaa 4080
tatttattaa tttttaaaat ttttgatatt tttaatttaa aacttatttt ttctttcttt 4140
ctattatgct tatttttgat attgaatagt agaagtgatt gatattaata attaatatct 4200
tttttaaaat atcaaagtct taaaaaaaat ataattaatt agttagttag ttacaaaata 4260
tggatagtta gcaacaaatc ggagatttat taactcatta cgaagctgag catttaatag 4320
aaaagttata aatcgttaac atagaagaat atggtagtta aatgtaaaat gctatttagt 4380
tttttagaca ttgttagtca tatcatcgca ctcacttaaa aattatcaat tatttataaa 4440
tagctggttc aagcaagacg aaattcttca aagactcaat atgcaagtat ttattttgcc 4500
attaatcaat tttattaaga aactatttca gcaaaagatc taaattttat ttcaaaaact 4560
cattttacta atatatgcat taaagattta tcaaaaactt ttattattat tatacattta 4620
ttttagatta gaaaaaagaa ttaatatgaa gtaatgagtt ttgaagttat ttgctttact 4680
ttaacttaaa aataatttaa tattatgttt tttcaattaa cattttatca atcaaaatgc 4740
ttaagaaatt aaatttaaag atatttttat atttcaaaaa tattttaggc acatgtcaat 4800
gcaatggtaa aaagtgatga gttcattatg gattctttag tgacttttga taaagtgaag 4860
attttgattt acgatttaat agaaacagaa atttggaaat agaaggtatt gcctttacta 4920
aaaaatcaca tgcttaaaat aaacacatat agaagctata ttgctgttta tcacgaagct 4980
gtagtctgta atttgctaga agtcattatg ttccatagaa ccgctgtcga ctcagctgat 5040
gaattc 5046

## Beschreibung

Die vorliegende Erfindung betrifft eine Triterpenoid-Cyclase (Tetrahymanol-Cyclase) aus Tetrahymena, ihre kodierende Nukleinsäure sowie ihre Herstellung und Verwendung.

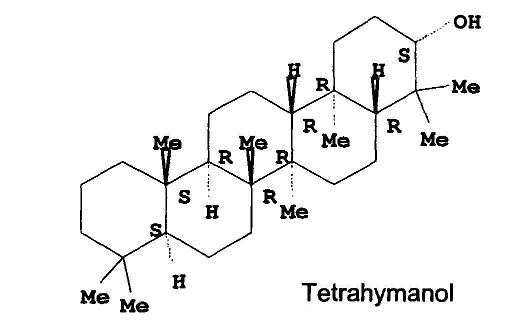

Die vorliegende Erfindung betrifft Nukleinsäuren aus Tetrahymena die für eine ciliatenspezifische Triterpenoid-Cyclase kodieren. Die erfindungsgemäßen Nukleotidsequenzen und die daraus abgeleitete Polypeptidsequenz zeigen dabei eine überraschend geringe Sequenzidentität zu bekannten Isoprenoid-Cyclasen. Ein weiterer Gegenstand der Erfindung ist die genomische Nukleotidsequenz der Triterpenoid-Cyclase, welche neben der kodierenden Sequenz nicht kodierende Nukleotidsequenzen wie Introns, Promoter und flankierende Sequenzen enthält. Die Erfindung betrifft ferner die Verwendung der Nukleinsäuren für eine kontrollierte Expression bzw. für eine gezielte Ausschaltung (Knock-out) oder Reprimierung dieses Gens. Durch die Kontrolle der Expression der Triterpenoid-Cyclase ist es möglich, den Gehalt an mehrfach ungesättigten Fettsäuren (sog. PUFA engl.: polyunsatturated fatty acids) zu modifizieren. Darüber hinaus kann durch die gezielte Ausschaltung (Knock-out) der Triterpenoid-Cyclase eine Anreicherung von Squalen, der Vorstufe verschiedener Triterpenoide erzielt werden. Squalen wird als Synthesebaustein für Terpene eingesetzt. Darüber hinaus findet Squalen, teilweise in modifizierter (z.B. hydrierten Form), Anwendung in der Dermatologie und Kosmetik, sowie in verschiedenen Derivaten in Haut- und Haarpflegemitteln. In einer weiteren Ausführung ermöglicht eine gezielte Überexpression der erfindungsgemäßen Nukleinsäuren die Herstellung von cyclischen Triterpenoiden, pentacyclischen Triterpenoiden z.B. Tetrahymanol oder Hopen, ebenfalls solche tetracyclischen Triterpenoiden wie z. B. Lanosterol oder Cycloartenol. Cyclische Triterpenoide finden in der Synthese von Steroidhormonen und Saponinen Verwendung. Aufgrund der guten Hautpenetration und Spreadingeigenschaften finden sie ebenfalls Anwendung in der Kosmetik und Dermatologie.

Die erfindungsgemäßen Nukleinsäuren sind erhältlich aus Ciliaten, vorzugsweise aus Tetrahymena, besonders bevorzugt aus Tetrahymena thermophila.

Die erfindungsgemäße Triterpenoid-Cyclase katalysiert bevorzugt die Bildung von Tetrahymanol aus Squalen durch eine direkte Cyclisierung von Squalen (Capsi et al. (1968) J. Am. Chem. Soc. 90: 3563-3564; Abe et al. (1993) Chem. Rev. 93: 2189-2206). Die Cyclase erkennt neben Squalen ebenfalls Oxidosqualen als Substrat (Abe & Rohmer (1994) J. Chem. Soc. Perkin Trans. 1:783-791). Neben pentacyclischen Triterpenoiden, kann die Squalen-Tetrahymanol-Cyclase ebenfalls die Bildung von tetracyclischen Triterpenoiden katalysieren (Abe & Rohmer (1991) J. Chem. Soc. Chem. Commun. 902-903). Tetrahymanol (oder Gammaceran-3-ol) gehört zur Stoffklasse der Isoprenoide, die sich von einer C₅-Einheit, dem Isopren, ableiten. Isoprenoide spielen eine wichtige Rolle z. B. als Phytohormone und Carotinoide, als Bestandteil von Chlorophyllen, Ubichinon, pflanzlichen Harzen, Ölen und Milchsäften. Als Steroidhormone z. B. besitzen sie wichtige Funktionen in Tieren. Die Bildung von Tetrahymanol kann z. B. bei Tetrahymena durch die Zugabe von Sterolen, z. B. Cholesterin, reprimiert werden (Conner et al. (1968) J. Protozool. 15: 600-605; Conner et al. (1969) J. Biol. Chem. 244:2325-2333).

Isoprenoide sind wichtige Bestandteile von bakteriellen und eukaryotischen Membranen. Wie Hopanoide und Sterole (z. B. Cholesterin) besitzt das pentacyclische Triterpenoid Tetrahymanol membranstabilisierende Eigenschaften (Conner et al. (1968; 1969), Poralla et al. (1980) FEBS Lett. 113: 107-110). Durch eine Einschränkung der Beweglichkeit der Fettsäurereste von Membranlipiden wird oberhalb der sogenannten Phasenübergangstemperatur ein kondensierender (Membran festigender) Effekt erreicht, unterhalb der Phasenübergangstemperatur wird die Fluidität von Membranen erhöht, indem die optimal dichte Packung der Fettsäurereste verhindert wird. Die Fluidität von Membranen hängt daneben vor allem von der Fettsäurezusammensetzung der Membranlipide ab. Die Fluidität von Membranen steigt mit dem Anteil an ungesättigten Fettsäuren. Bei wechselnden Temperaturen können Organismen die Fluidität ihrer Membranen z. B. über die Fettsäurezusammensetzung regulieren. Unterhalb der Phasenübergangstemperatur erhöhen Isoprenoide und ungesättigte Fettsäuren die Membranfluidität, sie wirken dabei synergistisch. Durch den Knock-out der Synthese der cyclischen Triterpenoide wird die Membranstabilität verändert. Die verringerte Membranfluidität kann durch einen erhöhten Anteil von PUFAs in der Membran ausgeglichen werden, d.h. der Gehalt an PUFAs kann durch einen Knock-out der Triterpenoid-Cyclase erhöht werden.

Die gezielte Modifizierung der Zusammensetzung des Fettsäurespektrums durch gentechnische Methoden für die kommerzielle Gewinnung spezieller Fettsäuren bzw. Ölen wird in Napier et al. (Curr. Opin. Plant Biol. (1999) 123-127), Murphy & Piffanelli (1998) Soc. Exp. Biol. Semin. Ser. 67 (Plant Lipid Biosynthesis) 95-130) und Facciotti & Knauf (In: Adv. Photosynth. 6: Lipids in Photosynthesis: Structure, Function and Genetics. Siegenthaler & Murata (eds.) Kluwer Academic Publishers. Netherlands. (1998) 225-248) beschrieben. Die Modifizierung der Fettsäurezusammensetzung wird dabei vor allem durch Gene beschrieben, die unmittelbar an der Fettsäuresynthese beteiligt sind, wie z.B. Desaturasen. Dabei hat sich gezeigt, daß der Gehalt an PUFAs der durch gentechnische Methoden in transgenen Organismen für eine kommerzielle Nutzung erreicht werden kann, bisher nur sehr gering ist (Knutzon DS & Knauf VC (1998) Soc. Exp. Biol. Semin. Ser. 67: 287-304).

Durch die Ausschaltung oder Reprimierung der Triterpenoid-Cyclase und das daraus resultierende Fehlen von z.B. Tetrahymanol, kann die Fettsäurezusammensetzung beeinflußt werden. Die veränderte Membranfluidität kann dabei durch vermehrte eigene Produktion des Gehalts an ungesättigten Fettsäuren ausgeglichen werden.

Obwohl die Triterpenoid-Cyclase aus Tetrahymena bereits bekannt ist und auch bereits gereinigt worden ist (Saar et al. (1991) Biochem. Biphys. Acta, Vol. 1075, No. 1), war es bisher nicht möglich die Triterpenoid-Cyclase aus Tetrahymena zu klonieren (Dissertationsschrift von Michal Perzl (1996) an der Fakultät für Biologie der Eberhard-Karls-Universität Tübingen). Weder durch Ansequenzieren des gereinigten Proteins, noch über die Konstruktion degenerierter Primer, noch über Hybridisierung mit heterologen Sonden war es bisher möglich die Sequenz der Triterpenoid-Cyclase zu ermitteln oder zu klonieren.

Aufgabe der vorliegenden Erfindung ist es daher, eine Nukleinsäure aus Tetrahymena bereitzustellen, welche für ein Polypeptid mit der Aktivität einer Triterpenoid-Cyclase kodiert. Eine weitere Aufgabe ist die kontrollierte Expression oder Ausschaltung (hinreichende Hemmung) der Triterpenoid-Cyclase in einem Wirtsorganismus, vorzugsweise in Tetrahymena selbst, zwecks Erhöhung des Gehalts an PUFAs, insbesondere zur Anreicherung von GLA, und zwar mittels wirtseigener Produktion von PUFA zur Wiederherstellung der Membranstabilität.

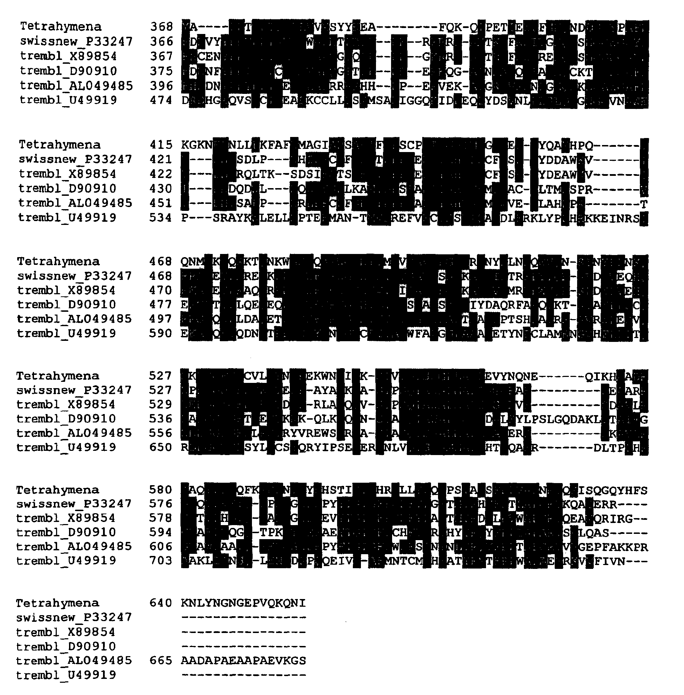

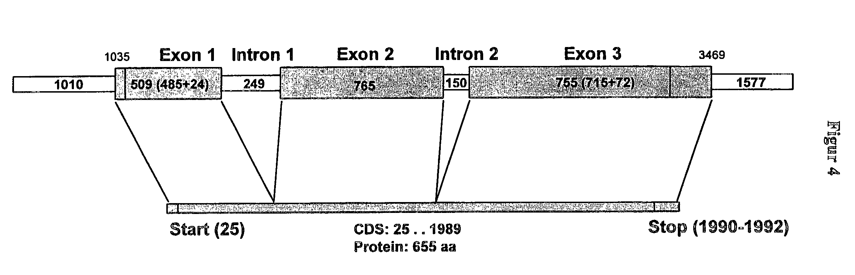

Gegenstand der vorliegenden Erfindung sind daher Nukleinsäuren gemäß SEQ ID Nr. 11 und 13 kodierend für eine Triterpenoid-Cyclase mit einer Aminosäuresequenz gemäß SEQ ID Nr. 12 oder eine funktionelle Variante davon, und Teile davon mit mindestens 8 Nukleotiden, vorzugsweise mit mindestens 15 oder 20 Nukleotiden, insbesondere mit mindestens 100 Nukleotiden, vor allem mit mindestens 300 Nukleotiden (nachfolgend "erfindungsgemäße Nukleinsäure(n)" genannt). Die Nukleinsäure gemäß SEQ ID Nr. 11 stellt dabei die proteinkodierende (cDNA) Sequenz dar. Die Nukleinsäure gemäß SEQ ID Nr. 13 stellt die genomische Sequenz der Triterpenoid-Cyclase dar, welche neben der kodierenden Sequenz auch nicht kodierende Nukleinsäuresequenzen wie Introns, Promoter und flankierende Sequenzen (UTR etc.) enthält (siehe Figur 4) und ein Gen ist.

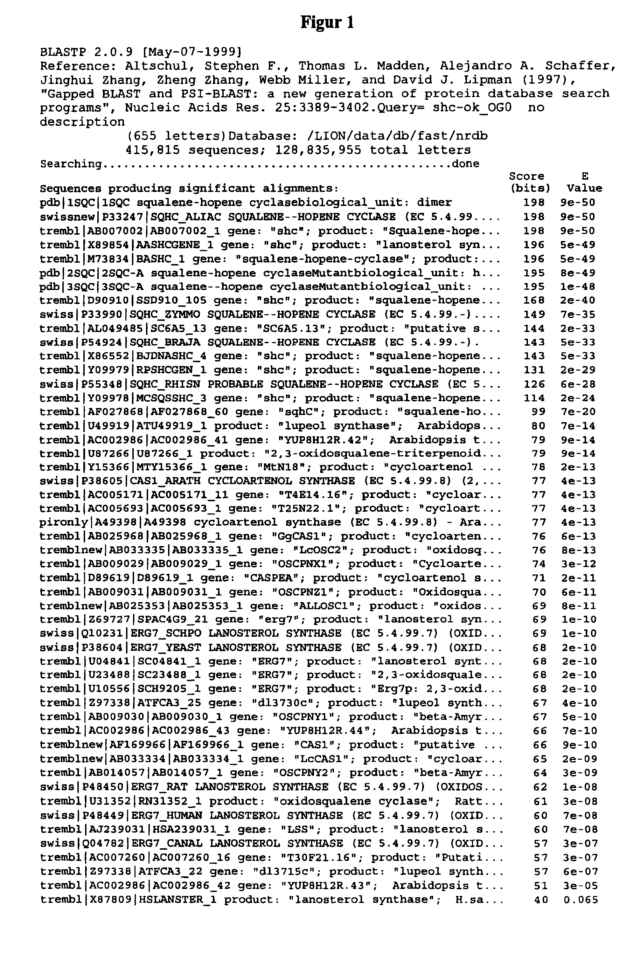

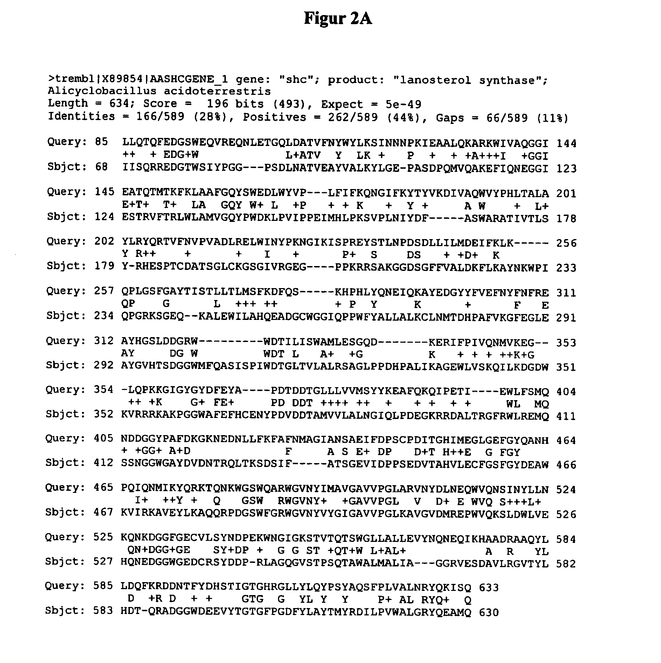

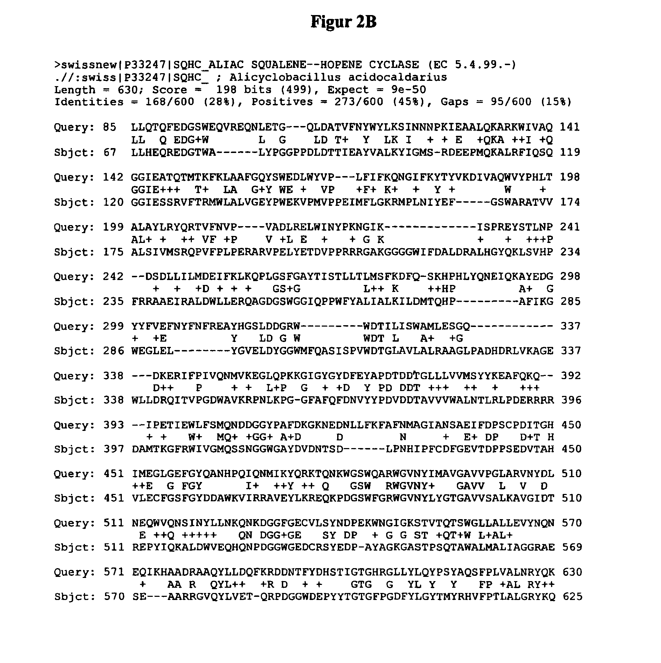

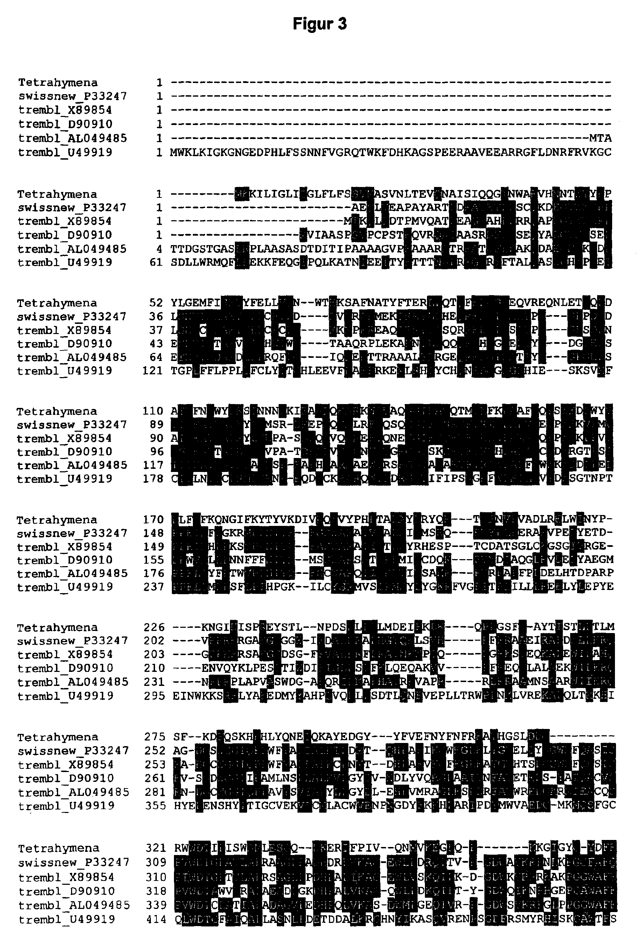

Die vollständige Nukleinsäure gemäß SEQ ID Nr. 11 kodiert für ein Protein mit 655 Aminosäuren und einer theoretischen molekularen Masse von 76,02 kDa. Die Sequenzanalysen gemäß der vorliegenden Erfindung bestätigen, daß es sich bei der Nukleinsäure um eine Nukleinsäure handelt, die für eine pentacyclische Triterpenoid-Cyclase aus Tetrahymena kodiert. Durch einen Homologievergleich konnte die aus der Nukleinsäuresequenz (SEQ ID Nr. 11) abgeleitete Proteinsequenz gemäß SEQ ID Nr. 12 als eine Triterpenoid-Cyclase identifiziert werden. Für den Homologievergleich wurde die Funktion BLASP (Altschul et al. 1997. Nucleic Acids Res. 25:3389-3402) benutzt. Als homologe Proteine wurden Isoprenoid-Cyclasen (z. B. Squalene-Hopen-Cyclasen, Lanosterol-, Cycloartenol-Synthase) aus Datenbanken identifiziert (siehe Figur 1). Die bekannten Triterpenoid-Cyclasen weisen dabei maximal eine Identität von 28% zu der erfindungsgemäßen Polypeptidsequenz auf (siehe Figur 2). Ein Multiples Alignment verschiedener bekannter Isoprenoid-Cyclasen mit der erfindungsmäßen Polypeptidsequenz ist in Figur 3 dargestellt. Die Homologien finden sich insbesondere in konservierten Domänen. Dazu gehört das sogenannten QW-Motiv (K/R X₂₋₃ F/Y/W L X₃ Q X₂₋₅ G X W; Poralla et al. (1994) Trends Biochem. Sci. 19: 157-158; Poralla (1994) Bioorg. Med. Chem. Lett. 4:285-290) das 7-8 mal in Squalen-Hopen-Cyclasen und 7 mal in Oxidosqualen-Cyclasen vorkommt. Das erfindungsgemäße Polypeptidsequenz weist sieben solcher QW-Motive auf, die jedoch deutlich weniger stark konserviert sind als bei anderen bekannten Triterpenoid-Cyclasen. Ein weiteres konserverties Motiv ist das Aspartat-reiche Motiv (D V/L D D T A; Perzl et al. (1997) Microbiology 143:1235-1242), das ebenfalls weniger gut konserviert in der erfindungsgemäßen Polypeptidsequenz an homologer Position vorkommt und zwar D T D D T G. Ähnliche Aspartat-reiche Motive wurden auch in anderen Enzymen der Isoprenoid-Biosynthese gefunden (Ashby et al. (1990) in: Molecular Biology of Atheriosclerosis, 27-34. Ed. AD Attie, Amsterdam, Elsevier). Obwohl die erfindungsgemäße Polypeptidsequenz sich als Triterpenoid-Cyclase identifizieren läßt, unterscheidet sie sich wesentlich von anderen Cyclasen. Die Identität der erfindungsgemäßen Polypeptidsequenz zu bekannten Cyclasen ist insgesamt überraschend gering.

In einer bevorzugten Ausführungsform ist die erfindungsgemäße Nukleinsäure eine DNA oder RNA, vorzugsweise eine doppelsträngige DNA, und insbesondere eine DNA mit einer Nukleinsäuresequenz bzw. eine funktionelle Variante der Nukleinsäuresequenz gemäß SEQ ID Nr. 11 oder der genomischen Nukleinsäuresequenz gemäß SEQ ID Nr. 13.

Unter dem Begriff"funktionelle Variante" versteht man gemäß der vorliegenden Erfindung eine Nukleinsäure, die funktionell mit der Triterpenoid-Cyclase aus Tetrahymena verwandt ist, beispielsweise andere pentacyclische Triterpenoid-Cyclasen, oder allelische oder degenerierte Varianten.

Im weiteren Sinne versteht man unter dem Begriff"Varianten" gemäß der vorliegenden Erfindung Nukleinsäuren, die eine Homologie, insbesondere eine Sequenzidentität von ca. 60%, vorzugsweise von ca. 75%, insbesondere von ca. 90% und vor allem von ca. 95% aufweisen. Hierbei ist der Hybridisierungsgrad ebenfalls heranzuziehen.

Darüberhinaus umfaßt die Erfindung auch funktionelle Varianten der Nukleinsäure, in der Basen in der Weise ausgetauscht werden, daß die dadurch kodierte Proteinsequenz nicht verändert wird. Aufgrund des ungewöhnlichen Codongebrauchs von Ciliaten (Wuitschick & Karrer (1999) Analysis of genomic G + C content, codon usage, initiator codon context and translation termination sites in Tetrahymena thermophila. J. Eukaryot. Microbiol. 1999 46(3):239-47) muß die beschriebene DNA-Sequenz für die Expression in anderen Systemen angepaßt werden. Zum einen betrifft dies den Austausch der Codons TAA und TAG, welche in Ciliaten Glutamin kodieren und in den meisten anderen Systemen Stop-Codons sind, in CAA und CAG. Darüber hinaus kann durch die Anpassung der Sequenz an die jeweilige besondere Codonpräferenz (sog. Codon-usage) verschiedener Systeme, die Expression für diese Systeme optimiert werden. Die Modifizierung der Nukleinsäuresequenz kann in dem Fachmann bekannter Weise durch einen gezielten Austausch von Basen durchgeführt werden. Alternativ kann die Sequenz aus künstlich hergestellten Oligonukleotiden zusammengesetzt werden. Die Anpassung der Sequenz kann z. B. auf der Basis der bekannten Codongebrauch-Tabellen (z. B. im Internet: Codon usage tabulated from Genbank: http://www.dna.affrc.go.jp/∼nakamura/CUTG.html) der bevorzugten Expressionssysteme durchgeführt werden. Ebenfalls umfaßt die vorliegende Erfindung Varianten von Nukleinsäuren, die nur Teile der Nukleinsäure enthalten.

Die Teile oder Fragmente der erfindungsgemäßen Nukleinsäuren können beispielsweise zur Herstellung einzelner Epitope, als Sonden zur Identifizierung weiterer funktioneller Varianten oder als Antisense-Nukleinsäuren verwendet werden. Beispielsweise eignet sich eine Nukleinsäure aus mindestens ca. 8 Nukleotiden als Antisense-Nukleinsäure, eine Nukleinsäure aus mindestens ca. 15 Nukleotiden als Primer beim PCR-Verfahren, eine Nukleinsäure aus mindestens ca. 20 Nukleotiden für die Identifizierung weiterer Varianten und eine Nukleinsäure aus mindestens ca. 100 Nukleotiden als Sonde.

In einer bevorzugten Ausführungsform sind die erfindungsgemäßen Nukleinsäuren in der Weise abgewandelt, daß die proteinkodierende Sequenz deletiert oder gegen ein selektierbares Gen, z. B. eine Resistenz gegen Antibiotika, ausgetauscht ist. Mit Hilfe der derart abgewandelten Nukleinsäure kann beispielsweise durch eine homologe Rekombination das native Gen in einem Wirtsorganismus ausgeschaltet werden (Gen knock-out), mit dem Ziel die Synthese von cyclischen Triterpenoiden zu blockieren, wodurch die Fettsäurezusammensetzung in diesem Wirtsorganismus modifiziert werden kann. Darüber hinaus kann die Nukleinsäuresequenz dergestalt abgewandelt werden, daß die Aktivität des Enzyms reduziert oder aufgehoben wird, z. B. durch Deletionen, Insertionen oder Punktmutationen.

In einer weiteren, speziellen Anwendung ist eine gezielte Expression der Triterpenoid-Cyclase möglich, mit dem Ziel der Herstellung der Triterpenoid-Cyclase bzw. von Tetrahymanol oder anderen cyclischen Triterpenoiden mit Hilfe dieses Proteins in dem jeweiligen Wirtsorganismus. Dazu ist die erfindungsgemäße Nukleinsäure in einem Vektor, vorzugsweise in einem Expressionsvektor enthalten.

Die Expressionsvektoren können beispielsweise prokaryotische oder eukaryotische Expressionsvektoren sein. Beispiele für prokaryotische Expressionsvektoren sind für die Expression in E. coli z. B. der T7 Expressionsvektor pGM10 (Martin, 1996), welcher für einen N-terminalen Met-Ala-His6-Tag kodiert, der eine vorteilhafte Reinigung des exprimierten Proteins über eine Ni2+-NTA-Säule ermöglicht. Als eukaryotische Expressionsvektoren für die Expression in Saccharomyces cerevisiae eignen sich z.B. die Vektoren p426Met25 oder p426GAL1 (Mumberg et al. (1994) Nucl. Acids Res., 22, 5767), für die Expression in Insektenzellen z.B. Baculovirus-Vektoren wie in EP-B1-0127839 oder EP-B1-0549721 offenbart, und für die Expression in Säugerzellen z.B. SV40-Vektoren, welche allgemein erhältlich sind.

Die rekombinante Triterpenoid-Cyclase oder Teile davon wird durch Methoden der Proteinreinigung (z. B. Ausubel et al. 1995) isoliert. Weitere detaillierte Informationen über geeignete Vektoren finden sich z. B. in Sambrook et al. (1989), Goeddel, ed. (1990) Methods in Enzymology 185 Academic Press, and Perbal (1988) A Practical Guide to Molecular Cloning, John Wiley and Sons, Inc.. Solche Vektoren enthalten vorzugsweise Sequenzelemente, welche die Expression beeinflussen, wie Promotoren, Enhancer-Elemente, "upstream" aktivierende Sequenzen etc.. Für die Expression der erfindungsgemäßen Nukleinsäure(n) eignen sich sowohl induzierbare als auch konstitutive Promotoren, oder z. B. gewebespezifische Promotoren. Für die Expression in pflanzlichen Zellen eignet sich z. B. der "cauliflower mosaic virus" (CaMV) 35S Promoter (Restrepo et al. (1990) Plant Cell 2 987) oder z. B. Promotoren, die bei der Samenentwicklung aktiviert werden.

Vorzugsweise handelt es sich bei den benutzten Vektoren um Shuttle-Vektoren (Wolk et al. (1984) Proc. Natl. Acad. Sci. USA, 1561-1565 and Bustos et al. (1991) J. Bacteriol. 174, 7525-7533).

Im allgemeinen enthalten die Expressionsvektoren auch für die Wirtszelle geeignete regulatorische Sequenzen, wie z. B. den trp-Promotor für die Expression in E. coli (s. z.B. EP-B1-0154133), den ADH-2-Promotor für die Expression in Hefen (Russel et al. (1983), J. Biol. Chem. 258, 2674), den Baculovirus-Polyhedrin-Promotor für die Expression in Insektenzellen (s. z.B. EP-B1-0127839) oder den frühen SV40-Promotor oder LTR-Promotoren z.B. von MMTV (Mouse Mammary Tumour Virus; Lee et al. (1981) Nature, 214, 228).

Vektoren, welche die erfindungsgemäßen Nukleinsäuresequenz enthalten, können durch Infektion, Transfektion, Elektroporation, Partikelbeschuß und anderen Methoden in Zellen eingeschleust werden. Im Sinne dieser Erfindung bedeutet Transformation generell die Einbringung von Nukleinsäure in eine Wirtszelle (z. B. Sambrook et al. 1989, Potrykus I (1991) Annu. Rev. Plant Biol. Plant Mol. Biol. 42:205-225, Christou P (1993) Curr. Opp. Biotech. 4:135-141).

Ebenso sind Vektoren bevorzugt, welche die erfindungsgemäße Nukleinsäuren in funktioneller Kombination mit Promotoren, oder anderen regulativen Elementen, oder in Kombination mit einem weiteren Gen, bevorzugt einem Selektionsmarker, beispielsweise einer Antibiotika-Resistenz, welcher in einer Wirtszelle aktiv sind, enthalten. In einer bevorzugten Ausführung handelt es sich bei diesen regulativen Elementen um Nukleinsäuresequenzen, welche in Ciliaten, insbesondere Tetrahymena, funktionell aktiv sind.

In einer bevorzugten Ausführungsform wird die erfindungsgemäße Nukleinsäure unter Kontrolle eines starken Promotors (wie z. B dem Tetrahymena Tubulin-Promoter, Gaertig et al. 1999) in Tetrahymena selbst exprimiert. Die Transformation kann vorzugsweise nach Gaertig et al. 1999 Nature Biotech. 17: 462-465 (oder z. B. nach Gaertig & Gorovsky (1992) Proc. Natl. Acad. Sci. USA 89:9196-9200) beschriebenen Methoden erfolgen. Als regulative Elemente für die Expression können z. B. die Promotoren von α- oder β-Tubulin aus Tetrahymena thermophila benutzt werden. Die transformierten Tetrahymena werden in selektiven Medien identifiziert, angereichert und kultiviert. Durch die Überexpression der erfindungsgemäßen Nukleinsäuren können cyklische Triterpenoide, bevorzugt pentacyklische Triterpenoide, besonders bevorzugt Tetrahymanol hergestellt werden.

Die erfindungsgemäßen Nukleinsäuren können beispielsweise chemisch anhand der in SEQ ID Nr. 11 und 13 offenbarten Sequenzen oder anhand der in SEQ ID Nr. 12 offenbarten Peptidsequenz unter Heranziehen des genetischen Codes z.B. nach der Phosphotriester-Methode synthetisiert werden (s. z.B. Uhlman, E. & Peyman, A. (1990) Chemical Reviews, 90, 543, No. 4). Eine weitere Möglichkeit, die erfindungsgemäßen Nukleinsäuren in die Hand zu bekommen, ist die Isolierung aus einer geeigneten Genbank, von einem Organismus, der Isoprenoid-Cyclase Aktivität besitzt, anhand einer geeigneten Sonde (s. z. B. Sambrook, J. et al. (1989) Molecular Cloning. A laboratory manual. 2nd Edition, Cold Spring Harbor, New York). Als Sonde eignen sich beispielsweise einzelsträngige DNA-Fragmente mit einer Länge von ca. 100 bis 1000 Nucleotiden, vorzugsweise mit einer Länge von ca. 200 bis 500 Nucleotiden, insbesondere mit einer Länge von ca. 300 bis 400 Nucleotiden, deren Sequenz aus der Nukleinsäuresequenz gemäß SEQ ID Nr. 11 oder 13 abgeleitet werden kann.

Ein weiterer Gegenstand der vorliegenden Erfindung ist ebenfalls das Polypeptid selbst mit einer Aminosäuresequenz gemäß SEQ ID Nr. 12 oder einer funktionellen Variante davon, und Teile davon mit mindestens sechs

Aminosäuren, vorzugsweise mit mindestens 12 Aminosäuren, insbesondere mit mindestens 65 Aminosäuren und vor allem mit mindestens 150 Aminosäuren (nachfolgend "erfindungsgemäßes Polypeptid" genannt). Beispielsweise kann ein ca. 6-12, vorzugsweise ca. 8 Aminosäuren-langes Polypeptid ein Epitop enthalten, das nach Kopplung an einen Träger zur Herstellung von spezifischen poly- oder monoklonalen Antikörper dient (siehe hierzu z. B. US 5,656,435). Polypeptide mit einer Länge von mindestens ca. 65 Aminosäuren können auch direkt ohne Träger zur Herstellung von poly- oder monoklonalen Antikörper dienen.

Unter dem Begriff"funktionelle Variante" im Sinne der vorliegenden Erfindung versteht man Polypeptide, die funktionell mit dem erfindungsgemäßen Peptid verwandt sind, d.h. eine Triterpenoid-Cyclase Aktivität aufweisen.

Im weiteren Sinne versteht man darunter auch Polypeptide, die eine Sequenzhomologie, insbesondere eine Sequenzidentität von ca. 70%, vorzugsweise von ca. 80%, insbesondere von ca. 90%, vor allem von ca. 95% zu dem Polypeptid mit der Aminosäuresequenz gemäß SEQ ID Nr. 12 haben.

Ferner zählen hierzu auch Deletion des Polypeptids im Bereich von ca. 1 - 60, vorzugsweise von ca. 1 - 30, insbesondere von ca. 1 - 15, vor allem von ca. 1 - 5 Aminosäuren. Beispielsweise kann die erste Aminosäure Methionin fehlen, ohne daß die Funktion des Polypeptids wesentlich verändert wird. Daneben zählen hierzu auch Fusionsproteine, die die oben beschriebenen erfindungsgemäßen Polypeptide enthalten, wobei die Fusionsproteine selbst bereits die Funktion einer Triterpenoid-Cyclase haben oder erst nach Abspaltung des Fusionsanteils die spezifische Funktion bekommen können. Vor allem zählen hierzu Fusionsproteine mit einem Anteil von insbesondere nicht-ciliaten Sequenzen von ca. 1 - 200, vorzugsweise ca. 1 - 150, insbesondere ca. 1 - 100, vor allem ca. 1 - 50 Aminosäuren. Beispiele von nicht-Ciliaten Peptidsequenzen sind prokaryotische Peptidsequenzen, z.B. aus der Galactosidase von E. coli oder ein sogenannter Histidin-Tag, z.B. ein Met-Ala-His6-Tag. Ein Fusionsprotein mit einem sogenannten Histidin-Tag eignet sich besonders vorteilhaft zur Reinigung des exprimierten Proteins über Metallionen-haltige Säulen, beispielsweise über eine Ni2+-NTA-Säule. "NTA" steht für den Chelator "nitrilotriacetic acid" (Qiagen GmbH, Hilden).

Die Teile des erfindungsgemäßen Polypeptids repräsentieren beispielsweise Epitope, die spezifisch von Antikörpern erkannt werden können.

Das erfindungsgemäße Polypeptid kann beispielsweise durch Expression der erfindungsgemäßen Nukleinsäure in einem geeigneten Expressionssystem, wie oben bereits beschrieben, nach dem Fachmann allgemein bekannten Methoden hergestellt werden. Als Wirtszellen eignen sich beispielsweise die E. coli Stämme DH5, HB101 oder BL21, der Hefestamm Saccharomyces cerevisiae, die Insektenzelllinie Lepidopteran, z.B. von Spodoptera frugiperda, oder tierische Zellen wie COS, Vero, 293 und HeLa, die alle allgemein erhältlich sind.

Insbesondere die genannten Teile des Polypeptids können auch mit Hilfe der klassischen Peptidsynthese (Merrifield-Technik) synthetisiert werden. Sie eignen sich insbesondere zur Gewinnung von Antiseren, mit deren Hilfe geeignete Genexpressionsbanken durchsucht werden können, um so zu weiteren funktionellen Varianten des erfindungsgemäßen Polypeptids zu gelangen.

Ein weiterer Gegenstand der vorliegenden Erfindung bezieht sich daher auf ein Verfahren zur Herstellung eines erfindungsgemäßen Polypeptids, wobei eine erfindungsgemäße Nukleinsäure in einer geeigneten Wirtszelle exprimiert und gegebenenfalls isoliert wird.

Ein anderer Gegenstand der vorliegenden Erfindung bezieht sich auch auf Antikörper, die mit dem erfindungsgemäßen Polypeptid spezifisch reagieren, wobei die oben genannten Teile des Polypeptids entweder selbst immunogen sind oder durch Koppelung an geeignete Träger, wie z.B. bovines Serumalbumin, immunogen gemacht bzw. in ihrer Immunogenität gesteigert werden können.

Die Antikörper sind entweder polyklonal oder monoklonal. Die Herstellung, die auch einen Gegenstand der vorliegenden Erfindung darstellt, erfolgt beispielsweise nach allgemein bekannten Methoden durch Immunisieren eines Säugetiers, beispielsweise eines Kaninchens, mit dem erfindungsgemäßen Polypeptid oder den genannten Teilen davon, gegebenenfalls in Anwesenheit von z. B. Freund's Adjuvant und/oder Aluminiumhydroxidgelen (s. z.B. Diamond, B.A. et al. (1981) The New England Journal of Medicine, 1344). Die im Tier aufgrund einer immunologischen Reaktion entstandenen polyklonalen Antikörper lassen sich anschließend nach allgemein bekannten Methoden leicht aus dem Blut isolieren und z.B. über Säulenchromatographie reinigen. Bevorzugt wird eine Affinitätsreinigung der Antikörper, bei der beispielsweise das C-terminale Triterpenoid-Cyclase an eine NHS-aktivierte HiTrap-Säule gekoppelt wurde.

Monoklonale Antikörper können beispielsweise nach der bekannten Methode von Winter & Milstein (Winter, G. & Milstein, C. (1991) Nature, 349, 293) hergestellt werden.

In einer bevorzugten Anwendung werden die erfindungsgemäßen Nukleinsäuren zur Ausschaltung der Triterpenoid-Cyclase in einem Wirtsorganismus, bevorzugt Tetrahymena, benutzt, mit dem Ziel den Gehalt von PUFA, inbesondere GLA in diesem Wirtsorganismus zu erhöhen. Die Ausschaltung kann durch homologe Rekombination mit einer Nukleinsäure gemäß SEQ ID Nr. 11 oder 13 erfolgen, die dergestalt abgewandelt ist, daß beispielsweise die proteinkodierende Sequenz gegen ein selektierbares Gen, z. B. eine Antibiotikaresistenz, ausgetauscht ist (Gaertig et al. (1994) Nucl. Acids Res. 22:5391-5398; Kahn et al. (1993) PNAS 90:9295-9299)). Neben dem knock-out durch homologe Rekombination (Galli-Taliadoros (1995) J. Immunol. Methods 181(1): 1-15) besteht die Möglichkeit durch Einsatz der Antisense-Technik (Atkins et al. (1994) Biol. Chem. Hoppe. Seyler 375(11): 721-729), oder auch durch die Antisense-Ribosomen-Technik (Sweeney et al. (1996) Proc. Natl. Acad. Sci. USA 93(16): 8518-8523) die Aktivität der Triterpenoid-Cyclase zu reduzieren.

Mit Hilfe der erfindungsgemäßen Nukleinsäuren, kodierend für eine ciliatenspezifische Triterpenoid-Cyclase aus Tetrahymena, können transgene Organismen, bevorzugt Tetrahymena selbst, erzeugt werden, welche einen erhöhten Gehalt an ungesättigte Fettsäuren enthalten. Die Erfindung findet Anwendung in der kommerziellen Produktion von PUFAs, insbesondere von GLA. In einer besonderen Ausführungsform kann durch die Ausschaltung oder Reduzierung der Tetrahymaol-Cyclase Aktivität in Kombination mit der funktionellen Überexpression von Fettsäure-Desaturasen der GLA-Gehalt in GLA-produzierenden Organismen wie Tetrahymena erhöht werden.

In einer bevorzugten Ausführungsform wird ein Wirtsorganismus, bevorzugt Tetrahymena, mit der erfindungsgemäßen Nukleinsäure oder den oben beschriebenen Konstrukten, transformiert (Gaertig et al. (1999) Nature Biotech. 17: 462-465, Gaertig & Gorovsky (1992) Proc. Natl. Acad. Sci. USA 89:9196-9200, Gaertig et al. (1994) Nucl. Acids Res. 22:5391-5398; Kahn et al. (1993) Proc. Natl. Acad. Sci. USA 90:9295-9299).

Die transformierten Tetrahymena werden in selektiven Medien identifiziert, angereichert und kultiviert. Aus den Zellen können die Lip(o)ide nach Standardmethoden isoliert werden (z.B. Dahmer et al., (1989) Journal of American Oil Chemical Society 66, 543). Die Methylesther der Fettsäuren können durch Gaschromatographie analysiert werden.

Die in der vorliegenden Erfindung beschriebenen isolierte Nukleinsäuren oder Teile davon können ebenfalls zur Isolierung verwandter Gene aus anderen Organismen, insbesondere z. B. aus anderen Protozoen bzw. Protisten (systematisiert nach Cavalier Smith, T. (1995), Cell cycles diplokaryosis and the archezoan origin of sex, Arch. Protistenk., 145 (3-4) 189-207), insbesondere Ciliaten, benutzt werden. Für die Isolierung homologer Gene kann die erfindungsgemäße Nukleinsäure oder Teile davon als markierte Probe eingesetzt werden. Durch die Hybridisierung der Probe an isolierte Nukleinsäure aus anderen Organismen können homologe Nukleinsäure-Sequenzen identifiziert und isoliert werden. Die Markierung der Probe kann in einer für den Fachmann bekannten Weise durchgeführt werden (Ausubel, Sambrook (supra)). Für die Markierung der Probe eignen sich z.B. radioaktive Nukleotide oder Nukleotide die mit detektierbaren Molekülen wie z.B. fluoresziernden Molekülen, Digoxigenin, Biotin, magnetischen Molekülen oder Enzymen verknüpft sind. Die Identifizierung und Isolierung homologer DNA-Sequenzen erfolgt durch den Nachweis der Markierung nach einer Hybridisierung der Probe an heterologe DNA. Für die Suche nach homologen Sequenzen bieten sich cDNA-Banken oder genomische Banken an. Für den Nachweis homologer Sequenzen eigenen sich darüber hinaus Southern und Northem-Blots. Alternativ kann homologe DNA, die mit der markierten Probe hybridisiert, auch durch selektives Zurückhalten der markierten Probe (z.B. mit Hilfe eines Magneten) isoliert werden.

Die Isolierung und Klonierung homologer Gene durch Kreuzhybridisierung kann durch dem Fachmann bekannte Methoden, wie sie z. B. in Ausubel et al. (1995, Current Protocols in Molecular Biology, Green Publishing Associates, New York) oder Sambrook et al. (1989, Molecular Cloning) beschrieben sind, erfolgen.

Auf der Basis der isolierten DNA-Sequenz und der dadurch kodierten

Proteinsequenz können zudem Oligonukleotide hergestellt werden, mit deren Hilfe homologe Nukleinsäuresequenzen mittels Polymerasekettenreaktion (PCR) amplifiziert werden können.

Eine weitere Möglichkeit zur Isolierung homologer Proteine besteht in der Detektion mit spezifischen Antikörpern gegen das durch die Sequenz der vorliegenden Erfindung kodierten Protein oder Teile davon (z. B. Peptid-Antikörper).

Für die Isolierung von genomischer DNA und von mRNA, sowie die Herstellung von genomischen und cDNA-Banken sind eine Vielzahl gut etablierter Methoden bekannt (z.B. in Sambrook et al. (1989) in Molecular Clonning: A Laboratory Manual, Cold Spring Harbor, NY).

### Die Erfindung wird durch die folgenden Figuren näher erläutert

- **Figur 1:**: Ergebnis eines BLASTP-Datenbank-Vergleichs der Proteinsequenz gemäß SEQ ID Nr. 12 mit Proteindatenbanken.
- **Figur 2:**: Alignment der Proteinsequenz gemäß SEQ ID Nr. 12 mit bekannten pentacyclischen Triterpenoid-Cyclasen.
- **Figur 3:**: Multiples Alignment der Polypeptidsequenz gemäß SEQ ID Nr. 12 aus Tetrahymena mit bekannten pentacyclischen Triterpenoid-Cyclasen.
- **Figur 4:**: Schematische Darstellung der Genstruktur der Triterpenoid-Cyclase aus Tetrahymena gemäß SEQ ID Nr. 11 und 9.

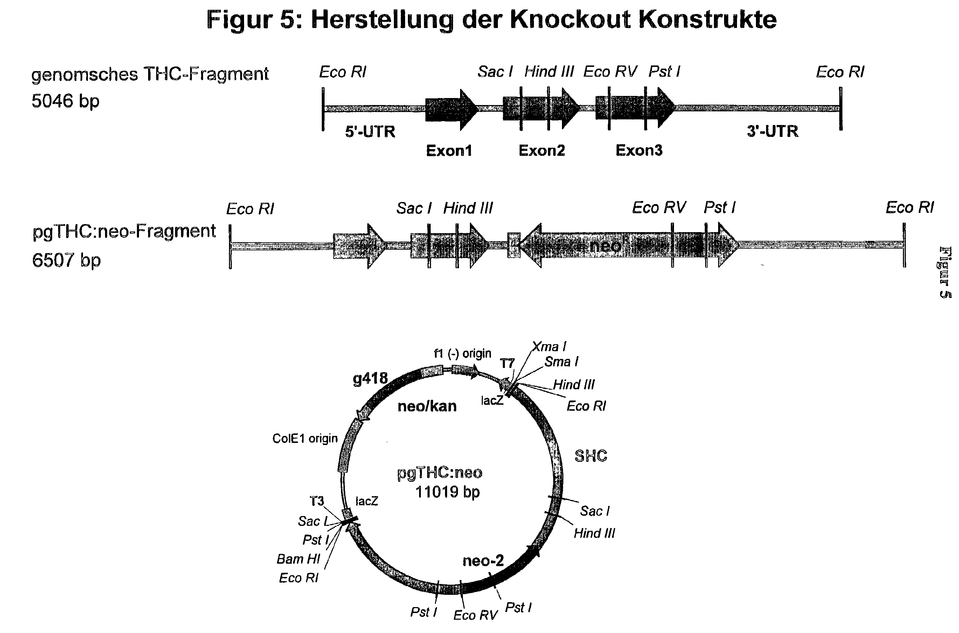

- **Figur 5:**: Herstellung der Triterpenoid-Knockout-Konstrukte.

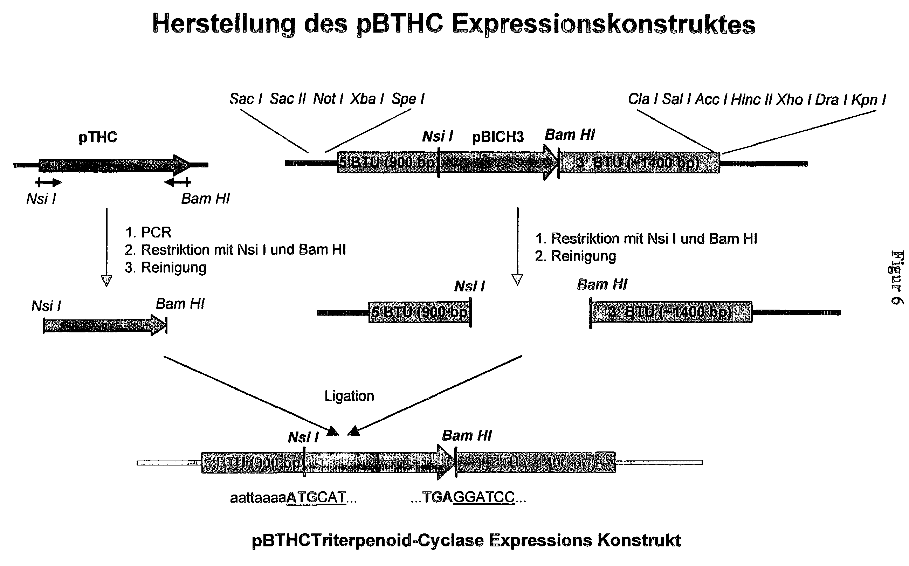

- **Figur 6:**: Herstellung des pBTHC Triterpenoid Expressionskonstruktes.

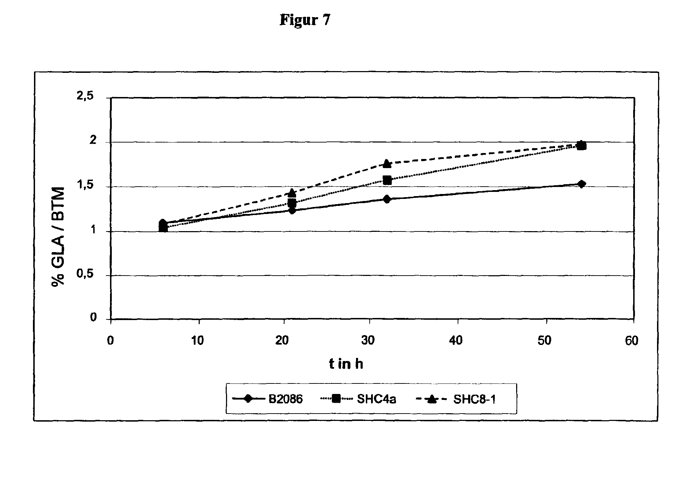

- **Figur 7:**: GLA in % der Biotrockenmasse der Transformanten im Vergleich zu Wildtyp-Tetrahymena.

### BEISPIELE

Die nachfolgenden Beispiele dienen zur Erläuterung der Erfindung, ohne die Erfindung auf diese Beispiele einzugrenzen.

### BEISPIEL 1:

### Organismen und Kultivierungsbedingungen

*Tetrahymena thermophila* (Stämme B1868 VII, B2086 II, B*VI, CU522, zur Verfügung gestellt von Dr. J. Gaertig, University of Georgia, Athens, GA, USA) wurden in modifiziertem SPP-Medium (2% Proteosepeptone, 0,1% Hefeextrakt, 0,2% Glucose, 0,003% Fe-EDTA (Gaertig et al. (1994) PNAS 91:4549-4553)) bzw. Magermilchmedium (2% Magermilchpulver, 0,5% Hefeextrakt, 1% Glucose, 0,003% Fe-EDTA) oder MYG-Medium (2% Magermilchpulver, 0,1% Hefeextrakt, 0,2% Glucose, 0,003% Fe-EDTA) mit Zusatz von Antibiotika-Lösung (100 U/ml Penicillin, 100 µg/ml Streptomycin und 0,25 µg/ml Amphotericin B (SPPA-Medium)) bei 30 °C in 50 ml Volumen in 250 ml Erlenmeyerkolben unter Schütteln (150 U/min) kultiviert.

Plasmide und Phagen wurden in *E. coli* XL1-Blue MRF', TOP10F' oder JM109 vermehrt und selektiert. Die Kultivierung der Bakterien erfolgte unter Standardbedingungen in LB- oder NZY-Medium, mit Antibiotika in Standardkonzentrationen (Sambrook et al. (1989) Molecular Cloning: A Laboratory Manual, Cold Spring Harbor Laboratory, Cold Spring, New York).

### BEISPIEL 2:

### Herstellung einer Tetrahymena thermophila cDNA-Bibliothek

Gesamt RNA aus *Tetrahymena thermophila* wurde nach der Guanidinthiocyanat/ Phenol/Chloroform Methode isoliert (Chomzynski & Sacchi (1987) Anal. Biochem. 161: 156-159). Aus der Gesamt-RNA wurde die mRNA mit Hilfe von Oligotex-dT-Kügelchen (Qiagen) isoliert. Die Synthese der cDNA erfolgte nach dem Stratagene ZAP Express cDNA Synthesis and Cloning Kit. Nach Ligation der EcoR I-Adapter und Verdau mit Xho I wurde die DNA über ein Agarosegel aufgetrennt und größenfraktioniert (S: 500 - 1500 bp, B: größer 1500 bp). Die DNA wurde aus dem Gel isoliert (Qiaquick Gel Extraktion Kit, QIAGEN) und in den Eco RI und Xho I geschnittenen ZAP Express Vektor ligiert. Die ligierte DNA wurde in-vitro in Phagen verpackt (Stratagene Gigapack III Gold) und die Phagen wurden in E. coli XL1-Blue MRF' vermehrt. Die S-cDNA-Bank enthielt ca. 5 x 10⁵ Klone mit einer durchschnittlichen Insertgröße von 1,1 kb, die B-cDNA-Bank enthielt ca. 6 x 10⁴ Klone mit einer durchschnittlichen Insertgröße von 2 kb.

### BEISPIEL 3:

Herstellung einer *Tetrahymena thermophila* genomischen DNA-Bibliothek Genomische DNA wurde mit Hilfe der Harnstoff-Methode (Gaertig et al. 1994) aus Tetrahymena isoliert und mit Eco R I geschnitten. Die geschnittene DNA wurde in einen ebenfalls Eco R I geschnittenen Lambda-Vektor (Zap Express, Stratagene) ligiert. Die weitere Durchführung entsprach der Vorgehensweise bei der cDNA-Bibliothek.

### BEISPIEL 4:

### RT-PCR mit Triterpenoid-Cyclase spezifischen Primern

Durch Sequenzvergleiche bekannter pentacyclischer Triterpenoid-Cyclasen konnten konservierte Bereiche identifiziert werden. Für die besonders stark konservierten Bereiche GSWF/YGR/SWGV/I und DGGWGE wurden unter Berücksichtigung des besonderen Ciliaten-Codon- bzw. *Tetrahymena*-Codon-Gebrauchs (Wuitschick JD, Karrer KM (1999) Analysis of genomic G + C content, codon usage, initiator codon context and translation termination sites in Tetrahymena thermophila. J. Eukaryot. Microbiol. 1999 46(3):239-47; CUTG, (Codon Usage Tabulated from Genbank):
http://www.dna.affrc.go.jp/∼nakamura/CUTG.html) PCR-Primer entworfen.

SEQ ID No 1: 5'-GGTTCNTGGTAYGGTAGATGGG-3';

SEQ ID No 2: 5'-TTCACCCCAACCACCATC-3'.

100 ng isolierte mRNA wurde für die Erststrangsynthese mit AMV-Reverser Transkriptase (Boehringer Mannheim) eingesetzt. Die Reaktion erfolgte nach dem Hersteller-Protokoll in 20 µl Volumen: 50 mM Tris-HCl (pH 8,5), 8 mM MgCl₂, 30 mM KCI, 1 mM DTT, 1 mM dNTPs, 2 pmol Oligo-dT-Ankerprimer SEQ ID No. 3: 5'- GACCACGCGTATCGATGTCGACT16V-3', 2 units AMV-Reverse Transkriptase, 60 min. bei 55 °C, anschließend 10 min 65 °C. 1/10 dieser Erststrangreaktion wurde für die PCR eingesetzt. Die PCR erfolgte in 25 µl Volumen mit: 1 x Qiagen HotStarTaq PCR-Puffer (QIAGEN), pH 8,7 (20 °C), je 10 pmol der genspezifischen Primer SEQ ID No.1 bzw. SEQ ID No.2, je 200 µM dNTPs, 1,5 mM MgCl₂, 1 unit HotStarTaq-Polymerase (Qiagen). Die PCR wurde unter folgenden Bedingungen durchgeführt: Anfangsdenaturierung bei 95 °C für 15 min, anschließend folgten 35 Zyklen mit jeweils 94 °C für 30 sec, 45 °C für 30 sec, 72 °C für 1 min. Abschließend 10 min 72 °C. PCR-Fragmente wurden durch T/A Klonierung (Invitrogen) in den Vektor pCR 2.1 ligiert und in E. coli TOP10F' (Invitrogen) vermehrt. Von positiven Klonen wurde Plasmid-DNA isoliert (Qiaprep Spin, QIAGEN) und sequenziert.

### BEISPIEL 5:

Isolierung der vollständigen Triterpenoid-Cyclase kodierenden cDNA Auf der Basis der so ermittelten Sequenz wurden neue Oligonukleotide für die PCR entworfen.

SEQ ID No. 4:	5'- CTGTTGGAGCTGTTGTACCAGG -3',

SEQ ID No. 5: 5'- CGTAATTGACTCTTGCTAAACCTGG -3'

Die Isolierung der vollständigen Triterpenoid-Cyclase kodierenden DNA erfolgte durch PCR mit diesen Primern in Kombination mit Vektor-spezifischen Primern (T3 und T7) aus der S-cDNA-Bank. Von der cDNA-Bank wurden 2 µl (10⁵ pfu/µl) für eine PCR (s.o) eingesetzt. Die PCR erfolgte abweichend von den oben angegebenen Bedingungen nach folgendem Protokoll: 15 min 95 °C Denaturierung, anschließend 35 Zyklen mit 20 sec. 94 °C, 20 sec 57 °C, 2 min. 72 °C. Abschließend 10 min 72 °C. Die PCR-Produkte wurden kloniert (s. o.) und sequenziert. Auf der Basis der so gewonnenen Sequenzinformationen wurde ein neuer Primer entworfen, der am 5'-Ende der cDNA-Sequenz lag.

SEQ ID No. 6:	5'- GCTAAAACTCTTTCATACATGAAGAAG -3'

Mit Hilfe dieses Primers in Kombination mit einem vektorspezifischen Primer konnte die vollständige cDNA durch PCR (PCR-Bedingungen s.o.) aus der cDNA-Bank amplifiziert und isoliert werden. Die so isolierte DNA wurde sequenziert. Die entsprechende cDNA-Sequenz ist in SEQ ID No. 11 angegeben.

Aus dieser cDNA-Sequenz kann die Proteinsequenz unter Berücksichtigung der besonderen Codon-Usage abgeleitet werden.

### BEISPIEL 6:

Isolierung der vollständigen genomischen Sequenz der Triterpenoid-Cyclase Durch Screening der genomischen Bank mit einem Digoxigenin markierten PCR-Produkt der Triterpenoid-Cyclase konnte ein Klon mit einem ca. 5000 bp großen DNA-Fragment isoliert werden. Durch in-vivo Excision wurde wurde aus dem isolierten Phagen ein Klon mit einem ca. 5000 bp Insert enthaltenden Plasmid erhalten (pgTHC). Durch Primerwalking wurde dieses Fragment vollständig sequenziert (SEQ ID Nr. 13). Durch Vergleich der ermittelten cDNA-Sequenz (SEQ ID Nr. 11) mit dieser Sequenz konnten Introns und flankierende Sequenzen identifiziert werden (s. Genstruktur, Figur 4).

### BEISPIEL 7:

### Herstellung von Triterpenoid-Cyclase Knock-out Konstrukten

Für die Herstellung des Knock-out Konstruktes wurde in die genomische Sequenz der Triterpenoid-Cyclase eine neo-Kassette aus dem Plasmid p4T2-1ΔH3 (Gaertig et al. (1994) Nucl. Acids Res. 22:5391-5398) inseriert. Dabei handelt es sich um das Neomycin-Resistenzgen unter der Kontrolle des Tetrahymena Histon H4-Promoters und der 3' flankierenden Sequenz des BTU2 Gens. Dieses Konstrukt vermittelt in Tetrahymena eine Resistenz gegenüber Paromomycin. Das Plasmid p4T2-1ΔH3 wurde mit Eco RV / Sma I geschnitten und das ca. 1,4 kb grosse Fragment der neo-Kassette wurde in das mit Eco RV geschnittene Plasmid pgTHC in die genomische Sequenz der Tetrahymena Triterpenoid-Cyclase ligiert. Dadurch wurde das Plasmid pgTHC::neo erzeugt. Bei einer erfolgreichen Transformation wurde durch homologe Rekombination das Gen für die Triterpenoid-Cyclase durch dieses Konstrukt ersetzt, wodurch eine Resistenz der Zellen gegenüber Paromomycin vermittelt wurde.

### BEISPIEL 8:

### Herstellung der Expressionskonstrukte pBTHC

Der Vektor pBICH3 (Gaertig et al. 1999 Nature Biotech. 17: 462-465) enthält die kodierende Sequenz des *Ichthyophthirius* I-Antigen (G1) Präprotein flankiert von den nicht kodierenden, regulativen Sequenzen des *Tetrahymena thermophila* BTU1-Gens. Ein modifiziertes Plasmid (pBICH3-Nsi) mit einer Nsi I Schnittstelle am Start (zur Verfügung gestellt von J. Gaertig, University of Georgia, Athens, GA, USA) wurde benutzt, um das Tetrahymanol-Cyclase Expressionskonstrukt pBTHC herzustellen. Dazu wurden mittels PCR Nsi I und Bam HI Schnittstellen am Start und Stop der kodierenden Sequenzen der Tetrahymanol-Cyclase von *Tetrahymena* eingefügt. Für die PCR wurden isolierte Plasmide, welche die vollständigen cDNA-Sequenzen der Tetrahymanol-Cyclase enthalten (pTHC) als Template eingesetzt. Die Primer SEQ ID No. 7: 5'- CTCTTTCATACATGCATAAGATACTCATAGGC-3' und SEQ ID No. 8:     5'-GGCTTGGATCCTCAAATATTTATTTTTATACAGG-3' erzeugten PCR-Produkte, welche die vollständige kodierende Sequenz der Tetrahymanol-Cyclase, flankiert von Nsi I und Bam HI Schnittstellen, enthielten. Die PCR-Produkte und das Plasmid pBICH3-Nsi wurden mit den Restriktionsenzymen Nsi I und Bam HI geschnitten, über ein Agarosegel gereinigt und ligiert (s. Abb. Plasmidkonstruktion). Die so entstandenen Expressionskonstrukte pBTHC enthielten die vollständige Triterpenoid-Cyclase kodierende Sequenz inseriert in einem korrekten Leseraster in die regulatorischen Sequenzen des BTU1 Gens. Für die Transformation von Tetrahymena wurden die Konstrukte durch einen Verdau mit den Restriktionsenzymen Xba I und Sal I linearisiert.

Bei einer erfolgreichen Transformation wurde durch homologe Rekombination das BTU1-Gen durch diese Konstrukte ersetzt, wodurch eine Resistenz der Zellen gegenüber Paclitaxel vermittelt wurde.

### BEISPIEL 9:

### Bestimmung des Fettsäurespektrums der Transformanten

Die Bestimmung des Fettsäurespektrums erfolgte mittels Gaschromatographen (HP GC 6890) mit Flammenionisationsdetektor (Hewlett-Packard Company, Wilmington, USA). Als Säule diente eine FFAP (Free Fatty Acid Phase) Permbond (Macherey & Nagel GmbH, Düren). Die Identifizierung der Fettsäuren erfolgte durch den Vergleich mit Retentionszeiten von Fettsäuremethylester-Standards. Auf der Basis der bekannten Konzentration des Standards konnte die Konzentration der Fettsäuren in den Proben bestimmt werden.

Zur Bestimmung des Fettsäurespektrums wurden die isolierten Transformanten in MYG-Medium (plus 10 µg/ml Cholesterol) bei 30 °C, 150 RPM für 24-96 h kultiviert. 50 ml der Kultur wurden bei 1500 g für 15 min zentrifugiert, der Überstand verworfen, das Pellet bei -80 °C eingefroren und anschliessend gefriergetrocknet. 50 mg der lyophillisierten Probe wurden eingewogen und mit 1 ml 20%iger methanolischer HCl und 1 ml methanolischer Standard-Lösung (1 mg/ml) versetzt. Zur Freisetzung der Fettsäuren und deren Umesterung in Fettsäuremethylester wurden die Proben im geschlossenen Reagenzglas zwei Stunden bei 60°C im Wasserbad gerührt und auf Raumtemperatur abgekühlt. Zum Neutralisieren der Probe wurde anschließend 1 ml wässrige, gesättigte Natriumhydrogencarbonat-Lösung zugegeben und vorsichtig gemischt. Durch Zugabe von n-Hexan wurden die Fettsäuremethylester extrahiert. Anschließende wurde der Ansatz kräftig durchmischt und durch Zentrifugation für 2 min. bei 4300 rpm eine Phasentrennung erreicht. Etwa 2/3 der oberen, organischen Phase wurden entnommen und 1 µl der Probe wurde auf die GC-Säule gespritz und analysiert.

**Tabelle 1:**

| | | | | | |
|---|---|---|---|---|---|
| GLA-Gehalt der Tetrahymena Triterpenoid-Cyclase-Knockout-Transformanten im Vergleich zum Tetrahymena-Wildstamm (B2086). Angegeben ist der prozentuale Anteil der GLA an der Biotrockenmasse (% GLA/BTM) und die prozentuale Differenz (% Differenz) der Transformanten gegenüber dem Wildstamm B2086. | | | | | |

| | **% GLA/BTM** | **% GLA/BTM** | ***% Differenz*** | ***%* GLA/BTM** | **% *Differenz*** |
|---|---|---|---|---|---|
| **t in h** | **B2086** | **AX004a** | ***AX004a*** | **AX081** | ***AX081*** |
| 6 | 1,10 | 1,03 | *- 6,8%* | 1,08 | *-1,9%* |
| 21 | 1,24 | 1,32 | *+ 6,5%* | 1,44 | *+ 16,1%* |
| 32 | 1,37 | 1,57 | *+ 14,6%* | 1,76 | *+ 28,5%* |
| 54 | 1,54 | 1,96 | *+ 27,3%* | 1,98 | *+ 28,6%* |

### BEISPIEL 10:

### Macronucleus-Transformation von Tetrahymena mit dem Tetrahymanol-Cyclase Expressionskonstrukt pBTHC

Für eine Transformation wurden 5 x 10⁶ *Tetrahymena thermophila* Zellen (CU522) eingesetzt. Die Kultivierung der Zellen erfolgte in 50 ml SPPA-Medium bei 30 °C in einem 250 ml Erlenmeyerkolben auf einem Schüttler bei 150 RPM bis zu einer Zelldichte von ca. 3-5 x 10⁵ Zellen/ml. Die Zellen wurden durch Zentrifugation (1200 g) für 5 min pelletiert und das Zellpellet wurde in 50 ml 10 mM Tris-HCl (pH 7,5) resuspendiert und wie vorher zentrifugiert. Dieser Waschschritt wurde wiederholt und die Zellen in 10 mM Tris-HCl (pH 7,5, plus Antibiotika) bei einer Zelldichte von 3 x 10⁵ Zellen/ml resuspendiert, in einen 250 ml Erlenmeyerkolben überführt und für 16-20 h ohne Schütteln bei 30 °C inkubiert (Hungerphase). Nach der Hungerphase wurde erneut die Zellzahl bestimmt, wie oben zentrifugiert und die Zellen mit 10 mM Tris-HCl (pH 7,5) auf eine Konzentration von 5 x 10⁶ Zellen/ml eingestellt. Ein ml der Zellen wurde für die Transformation benutzt. Die Transformation erfolgte mittels Mikropartikelbeschuss (s.u.). Zur Regeneration wurden die Zellen in SSPA-Medium aufgenommen und bei 30 °C ohne Schütteln im Erlenmeyerkolben inkubiert. Nach 3 h wurde Paclitaxel® in einer Endkonzentration von 20 µM zugegeben und die Zellen in Aliquots von 100 µl auf 96er Mikrotiterplatten überführt. Die Zellen wurden in einer feuchten, abgedunkelten Box bei 30 C inkubiert. Nach 2-3 Tagen konnten Paclitaxel resistente Klone identifiziert werden. Positive Klone wurden in frisches Medium mit 25 µM Paclitaxel überimpft. Durch Kultivierung der Zellen in steigender Paclitaxel-Konzentration (bis 80 µM) wurde ein komplettes "phenotypic assortment" (Gaertig & Kapler (1999)) erreicht.

Zur Analyse der Klone wurden ca. 4 ml Kulturen in SPPA mit Paclitaxel angezogen, DNA isoliert (Jacek Gaertig et al. (1994) PNAS 91:4549-4553) und durch PCR die in den BTU1-Locus integrierte DNA amplifiziert. Als Primer dienten die BTU1 spezifischen Primer SEQ ID No. 9: 5'-AAAAATAAAAAAGTTTGAAAAAAAACCTTC-3', ca 50 bp vor dem Startcodon liegend und SEQ ID No. 10:	5'-GTTTAGCTGACCGATTCAGTTC-3', 3 bp hinter dem Stopcodon liegend.

Die PCR-Produkte wurden ungeschnitten und mit Hind III, Sac I oder Pst I geschnitten auf einem 1%igen Agarosegel analysiert. Vollständiges "phenotypic assortment" wurde über RT-PCR mit den BTU1 spezifischen Primern (Gaertig & Kapler (1999)) überprüft.

### BEISPIEL 11:

### Micronucleus- und Macronucleus-Transformation von Tetrahymena mit dem Knock-out Konstrukt pgTHC::neo

Tetrahymena Stämme unterschiedlichen Paarungstyps (CU428 VII und B2086 II) wurden getrennt in SPPA-Medium bei 30 °C unter Schütteln (150 Upm) in einem Erlenmeyerkolben kultiviert. Bei einer Zelldichte von 3-5 x 10⁵ Zellen/ml wurden die Zellen 5 min bei Raumtemperatur zentrifugiert (1200 g). Die Zellen wurden dreimal mit 50 ml 10 mM Tris-HCl (pH 7,5) gewaschen und schließlich in 50 ml 10 mM Tris-HCl (pH 7,5) resuspendiert und mit Antibiotika-Lösung versetzt. Die Zellen wurden in einem Erlenmeyerkolben bei 30 °C ohne Schütteln inkubiert. Nach ca. 4 h wurden beide Kulturen erneut gezählt und mit 10 mM Tris-HCl (pH 7,5) auf 3 x 10⁵ Zellen/ml verdünnt für weitere 16-20 h bei 30 °C inkubiert. Nach dieser Hungerphase wurde von beiden Kulturen die gleiche (absolute) Zellzahl in einem 2-L Erlenmeyerkolben gemischt. Die Zellen wurden bei 30 °C inkubiert (Beginn der Konjugation) und nach 2 h wurde die Effizienz der Konjugation bestimmt. Für eine erfolgreiche Transformation sollten zu diesem Zeitpunkt ca. 30% der Zellen als Paare vorliegen.

Für die Micronucleus-Transformation wurden 3 h, 3,5 h, 4 h und 4,5 h nach Beginn der Konjugation jeweils 1 x 10⁷ konjugierende Zellen (5 x 10⁶ Paare) 5 min bei 1200 g zentrifugiert und das Zellpellet in 1 ml 10 mM Tris-HCl (pH 7,5) resuspendiert.

Für die Transformation der neuen Macronucleus-Anlagen wurden 11 h nach Beginn der Konjugation Zellen wie oben zentrifugiert und in Tris-HCl resuspendiert. Die Transformation erfolgte mittels Mikropartikelbeschuss (s.u.). Für die Kultivierung der Tetrahymanol-Cyclase Knockout-Mutanten wurde 10 µg/ml Cholesterol zum Medium zugegeben.

Durch Selektion auf Paromomycinresistenz konnten transformierte Zellen identifiziert werden. Bei der Transformation des Mikronucleus wurde 11 h nach Beginn der Konjugation Paromomycin (100 µg/ml Endkonzentration) zugegeben und die Zellen in Aliquots von 100 µl auf 96er Mikrotiterplatten verteilt. Die Zellen wurden in einer feuchten Box bei 30 °C inkubiert. Nach 2-3 Tagen konnten resistente Klone identifiziert werden. Echte Mikronucleus-Transformanten konnten anhand der Resistenz gegenüber 6-Methylpurin von Macronucleus-Transformanten unterschieden werden. Bei der Transformation des Macronucleus wurde ca. 4 h nach der Transformation Paromomycin (100 µg/ml Endkonzentration) zugegeben und die Zellen in Aliquots von 100 µl auf 96er Mikrotiterplatten verteilt. Die Zellen wurden in einer feuchten Box bei 30 °C inkubiert. Nach 2-3 Tagen konnten resistente Klone identifiziert werden. Positive Klone wurden in frisches Medium mit 120 µg/ml Paromomycin überimpft. Durch Kultivierung der Zellen in dieser hohen Paromomycin-Konzentration wurde nach einigen Generationen ein komplettes "phenotypic assortment" (Gaertig & Kapler (1999)) erreicht.

Durch Kreuzung der Micronucleus-Transformanten mit einem B*VI-Stamm konnten homozygote Knockout-Mutanten erzeugt werden (Bruns & Cassidy-Hanley, Methods in Cell Biology, Volume 62 (1999) 229-240).

### BEISPIEL 12:

### Biolistische Transformation (Mikropartikelbeschuss)

Die Transformation von *Tetrahymena thermophila* erfolgte mittels biolistischer Transformation, wie sie bei Bruns & Cassidy-Hanley (Methods in Cell Biology, Volume 62 (1999) 501-512); Gaertig et al. (1999) Nature Biotech. 17: 462-465) oder Cassidy-Hanley et al. ((1997) Genetics 146:135-147) beschrieben ist. Die Handhabung des Biolistic® PDS-1000/He Particle Delivery System (BIO-RAD) ist detailiert im zugehörigen Handbuch beschrieben.

Für die Transformation werden 6 mg Goldpartikel (0,6 µm; BIO-RAD) mit 10 µg linearisierter Plasmid DNA beladen (Sanford et al. (1991) Biotechniques 3:3-16; Bruns & Cassidy-Hanley (1999) Methods in Cell Biology, Volume 62: 501-512). Vorbereitung der Goldpartikel: 60 mg der 0,6 µm Goldpartikel (Biorad) wurden in 1 ml Ethanol resuspendiert. Dazu wurden die Partikel 3 mal für je 1-2 min auf einem Vortex kräftig gemixt. Anschliessend wurden die Partikel für 1 min zentrifugiert (10000 g) und der Überstand vorsichtig mit einer Pipette abgenommen. Die Goldpartikel wurden in 1 ml sterilem Wasser resuspendiert und wie oben zentrifugiert. Dieser Waschschritt wurde einmal wiederholt, die Partikel in 1 ml 50%igem Glycerol resuspendiert und in Aliquots zu 100 µl bei -20 °C gelagert.

Vorbereiten der Transformation: Die Macrocarrierhalter, Macrocarrier und Stopscreens wurden für mehrere Stunden in 100% Ethanol, die Rupture Disks in Isopropanol gelagert. Ein Macrocarrier wurde anschließend in den Macrocarrier-Halter eingesetzt und an der Luft getrocknet.

Beladung der Goldpartikel mit DNA: Alle Arbeiten erfolgten bei 4 °C. Goldpartikel, vorbereiteter Vektor, 2,5 M CaCl₂, 1 M Spermidine, 70% und 100% Ethanol wurdenauf Eis gekühlt. 10 µl der linearisierten Vektor-DNA (1 µg/ml) wurden zu 100 µl vorbereiteten Goldpartikeln gegeben und für 10 sec vorsichtig gevortext. Anschliessend wurden erst 100 µl 2,5 M CaCl₂ zugegeben, 10 sec gevortext und dann 40 µl 1 M Spermidine zugegeben und 10 min vorsichtig gevortext. Nach Zugabe von 200 µl 70%igem Ethanol wurden die Partikel für 1 min gevortext und dann 1 min bei 10000 g zentrifugiert. Das Pellet wurde in 20 µl 100%igem Ethanol resuspendiert, zentrifugiert und dann in 35 µl 100%igem Ethanol resuspendiert. Die so vorbereiteten Partikel wurden vorsichtig mit einer Pipette auf das Zentrum eines Macrocarriers gegeben. Der Macrocarrierer wurde anschliessend bis zur Transformation in einer Box mit hygroskopischen Silicagel gelagert.

Transformation: Ein ml der vorbereiteten Zellen (siehe oben) wurde in die Mitte eines mit 10 mM Tris-HCl (pH 7,5) angefeuchteten Rundfilters in einer Petrischale gegeben und in die unterste Einschubleiste der Transformationskammer des Biolistic® PDS-1000/He Particle Delivery Systems eingesetzt. Die Transformation erfolgte mit den vorbereiteten Goldpartikeln bei einem Druck von 900 psi (zwei 450 psi Rupture Disks) und einem Vacuum von 27 inches Hg in der Transformationskammer. Anschliessend wurden die Zellen sofort in einen Erlenmeyerkolben mit 50 ml SPPA-Medium überführt und bei 30 °C ohne Schütteln inkubiert.

## Patentansprüche

1. Nukleinsäure kodierend für eine Triterpenoid-Cyclase mit einer Aminosäuresequenz gemäß SEQ ID No. 12 oder eine funktionelle Variante davon, und Teile dieser Nukleinsäure aus mindestens 8 Nukleotiden, wobei SEQ ID No. 11 Teil des Anspruchs ist.

2. Nukleinsäure nach Anspruch 1, dadurch gekennzeichnet, daß die Nukleinsäure eine DNA oder RNA, vorzugsweise eine doppelsträngige DNA ist.

3. Nukleinsäure nach Anspruch 1 oder 2, dadurch gekennzeichnet, daß die Nukleinsäure aus einer DNA mit einer Nukleinsäuresequenz gemäß SEQ ID No. 13 erhältlich ist, wobei SEQ ID No. 13 Teil des Anspruchs ist.

4. Nukleinsäure nach Anspruch 3, dadurch gekennzeichnet, daß die Nukleinsäure eine oder mehrere nicht-kodierende Sequenzen enthält.

5. Nukleinsäure nach Anspruch 3 und 4, dadurch gekennzeichnet, daß die Nukleinsäure SEQ ID No. 13 ein Gen ist.

6. Antisense Nukleinsäure zu einer der Nukleinsäuren gemäß Anspruch 1-5.

7. Nukleinsäure nach einem der Ansprüche 1-6, dadurch gekennzeichnet, daß die Nukleinsäure in einem Vektor, vorzugsweise in einem Expressionsvektor, enthalten ist.

8. Verfahren zur Herstellung einer Nukleinsäure nach einem der Ansprüche 1-6, dadurch gekennzeichnet, daß die Nukleinsäure chemisch synthetisiert oder anhand einer Sonde aus einer Genbank isoliert wird.

9. Polypeptid mit einer Aminosäuresequenz gemäß SEQ ID No. 12 oder eine funktionelle Variante davon, und Teile davon mit mindestens 6 Aminosäuren.

10. Verfahren zur Herstellung eines Polypeptids gemäß Anspruch 9, dadurch gekennzeichnet, daß eine Nukleinsäure gemäß einem der Ansprüche 1-5 in einer geeigneten Wirtszelle / Wirtsorganismus exprimiert wird.

11. Verfahren nach Anspruch 10, dadurch gekennzeichnet, daß der Wirtsorganismus ausgewählt ist aus Protozoen, vorzugsweise Ciliaten.

12. Antikörper gegen ein Polypeptid gemäß Anspruch 9.

13. Verfahren zur Herstellung eines Antikörpers gemäß Anspruch 12, dadurch gekennzeichnet, daß ein Säugetier mit einem Polypeptid gemäß Anspruch 9 immunisiert und gegebenenfalls die entstandenen Antikörper isoliert werden.

14. Verwendung einer Nukleinsäure gemäß einem der Ansprüche 1-5 zum Auffinden von Varianten der Triterpenoid-Cyclase, dadurch gekennzeichnet, daß eine Genbank mit der genannten Nukleinsäure abgesucht und die gefundene Variante isoliert wird.

15. Verfahren zur Anreicherung von hochungesättigten Fettsäuren in einer Wirtszelle oder Wirtsorganismus, dadurch gekennzeichnet, daß mindestens eine kodierende Sequenz einer Nukleinsäure gemäß einer der Ansprüche 1-5 inaktiviert wird.

16. Verfahren nach Anspruch 15, dadurch gekennzeichnet, daß die kodierende Sequenz mittels einer Antisense Nukleinsäure gemäß Anspruch 5 inaktiviert wird.

17. Verfahren nach Anspruch 15, dadurch gekennzeichnet, daß die kodierende Sequenz mittels Deletion, Insertion, Mutation inaktiviert wird.

18. Verfahren nach Anspruch 17, dadurch gekennzeichnet, daß mindestens ein selektierbarer Marker in die kodierende Sequenz insertiert wird.

19. Verfahren nach Anspruch 13, dadurch gekennzeichnet, daß Squalen in der Wirtszelle angereichert wird.

20. Transgene Wirtszelle / Wirtsorganismus enthaltend mindestens eine Nukleinsäure gemäß Anspruch 1-5.

21. Wirtsorganismus nach Anspruch 20 ausgewählt aus Protozoen, vorzugsweise Ciliaten

22. Verwendung der Nukleinsäuren gemäß einer der Ansprüche 1-5 oder ein Polypeptid gemäß Anspruch 9 zur Herstellung von cyklischen Triterpenoiden, vorzugsweise pentacyklische Triterpenoide, besonders bevorzugt Tetrahymanol.
